## Europäisches Patentamt
## European Patent Office
## Office européen des brevets

(19)

(11) Veröffentlichungsnummer: **0 256 223**
**A1**

(12) **EUROPÄISCHE PATENTANMELDUNG**

(21) Anmeldenummer: **87107137.9**

(22) Anmeldetag: **18.05.87**

(51) Int. Cl.³: **C 12 N 15/00**
**C 07 H 21/04, C 12 N 5/00**
**A 01 H 1/00, C 12 N 9/10**

(30) Priorität: **19.05.86 US 864467**
**26.09.86 US 912755**

(43) Veröffentlichungstag der Anmeldung:
**24.02.88 Patentblatt 88/8**

(84) Benannte Vertragsstaaten:
**AT BE DE ES FR GB GR IT LU NL SE**

(71) Anmelder: **CIBA-GEIGY AG**
**Klybeckstrasse 141**
**CH-4002 Basel(CH)**

(72) Erfinder: **Helmer, Georgia**
**3508 Lexham Court**
**Apex North Carolina 27502(US)**

(72) Erfinder: **Duesing, John**
**Gatternweg 18**
**CH-4125 Riehen(CH)**

(72) Erfinder: **Rothstein, Steven**
**417 Overland Drive**
**Chapel Hill North Carolina 27514(US)**

(72) Erfinder: **Scarafia, Liliana**
**408 Colony Woods Drive**
**Chapel Hill North Carolina 27514(US)**

(72) Erfinder: **Chilton, Mary-Dell**
**10513 Winding Wood Trail**
**Raleigh North Carolina 27612(US)**

(72) Erfinder: **Lai, Hui-Chen Jean**
**445 Waupelani Drive**
**State College PA 16801(US)**

(72) Erfinder: **Tu, Chen-Pei David**
**115 Cherry Ridge Road**
**State College PA 16803(US)**

(74) Vertreter: **Zumstein, Fritz, Dr. et al,**
**Bräuhausstrasse 4**
**D-8000 München 2(DE)**

(54) **Herbizid-tolerante Pflanzen, enthaltend ein Glutathion-S-Transferase-Gen.**

(57) Die vorliegende Erfindung betrifft die Anwendung der rekombinanten DNA-Technologie für die Transformation von Pflanzen, die zu einer Uebertragung einer Herbizid-Resistenz führt.

Insbesondere betrifft die vorliegende Erfindung die Konstruktion und Verwendung eines rekombinanten DNA-Moleküls, das ein Glutathion-S-Transferase (GST)-Gen beinhaltet, das im Zuge der Expression in der Pflanze die enzymatische Aktivität der GST erhöht.

EP 0 256 223 A1

CIBA-GEIGY AG

Basel (Schweiz)

5-15883/+/CGC 1198/CIP

Herbizid-tolerante Pflanzen, enthaltend ein
Glutathion-S-Transferase-Gen

Die vorliegende Erfindung betrifft die Uebertragung von Herbizid-
toleranz auf Pflanzen unter Anwendung der rekombinanten DNA-
Technologie, wobei die besagte Herbizidtoleranz aus der Detoxifikation des entsprechenden Herbizids resultiert.

Die vorliegende Erfindung betrifft insbesondere die Herstellung und
Verwendung eines rekombinanten DNA-Moleküls, welches ein Glutathion-
S-Transferase (GST)-Gen umfasst und das bei der Expression in der
Pflanze die GST-Enzymaktivität in der Pflanze erhöht.

Die Glutathion-S-Transferasen (EC 2.5.1.18) gehören zu einer Klasse
von Enzymen, die bei der Detoxifikation von Xenobiotika eine
wichtige Rolle spielen. Diese Enzyme findet man bei den meisten
lebenden Organismen, einschliesslich Mikroorganismen, Pflanzen,
Insekten und höheren Tieren. Jedes der Glutathion-S-Transferase
(GST)-Enzyme innerhalb dieser Klasse unterscheidet sich zwar vom
anderen, aber alle diese Enzyme besitzen eine sich überlappende
Substratspezifität. Vgl.: Jakobi et al., "Rat Glutathione
S-transferases: Binding and Physical Properties," in Glutathione:
Metabolism and Function, ed. I. Arias and W. Jakoby (Raven Press,
New York, 1976); Reddy et al., "Purification and Characterization
of Individual Glutathione S-Transferase from Sheep Liver, "Archives
of Biochem. and Biophys., 224: 87-101 (1983).

Unter den zahlreichen GST-Funktionen ist die Katalyse der Konjugation von Glutathion mit elektrophilen Verbindungen von besonderem Interesse. H. Rennenberg, "Glutathione Metabolism and Possible Biological Roles in Higher Plants," Phytochemistry, 21: 2771-2781 (1982); vgl.: Meister and Tate, "Glutathione and Related Gamma-Glutamyl Compounds: Biosynthesis and Utilization," in Ann. Rev. Biochem., 45: 560-604 (1976). Zahlreiche Xenobiotika, darunter Herbizide, Pestizide und Isektizide gehören zu den elektrophilen Verbindungen. Im Verlauf der Konjugation des Glutathion und einer elektrophilen Verbindung reagiert die Sulfhydryl-Gruppe des Glutathion mit dem elektrophilen Zentrum dieser Verbindung, wobei das Glutathion als Nucleophil fungiert. Diese Konjugation wird von einem spezifischen GST-Enzym katalysiert. (Rennenberg, supra).

Für die Pflanzen ist diese Reaktion von besonderer Bedeutung, da sie eine Möglichkeit zur Detoxifikation von Xenobiotika bietet. Die konjugierte, elektrophile, xenobiotische Verbindung wird dabei wasserlöslich und ist somit für die Pflanze nicht mehr toxisch.

Es wäre daher wünschenswert, Pflanzen zu entwickeln, die gegenüber Herbiziden tolerant sind, indem man unter Verwendung gentechnologischer Methoden die Enzymaktivität der Glutathion-S-Transferase in besagten Pflanzen deutlich erhöht. Auf diese Weise wäre es möglich, Pflanzen mit einer Toleranz gegenüber Herbiziden auszustatten.

Gegenstand der vorliegenden Erfindung sind rekombinante DNA-Moleküle, die eine Herbizid-Toleranz auf Pflanzen übertragen, wobei die Herbizide durch Produktion von Proteinen detoxifiziert werden. Zu den bekannten Detoxifikationsmechanismen gehören die Konjugation von Glutathion an elektrophile Verbindungen, eine Reaktion, die durch die Glutathion-S-Transferase katalysiert wird; die Konjugation von D-Aminosäure an 2,4-Dichlorphenoxyessigsäure (2,4-D) sowie die Hydroxylierung und Kohlenhydrat-Konjugation bei Sulfonylharnstoffen.

Insbesondere betrifft die vorliegende Erfindung herbizidtolerante Pflanzen, die mit einem rekombinanten DNA-Molekül transformiert sind, welches für ein Enzym kodiert, das für die Detoxifikation von Herbiziden verantwortlich ist. Weiterhin betrifft die vorliegende Erfindung in erster Linie rekombinante DNA-Moleküle, die durch eine Gen-Sequenz gekennzeichnet sind, welche für ein Glutathion-S-Transferase-Polypeptid kodiert, sowie herbizidtolerante transformierte Pflanzen, die eine erhöhte Glutathion-S-Transferase-Enzymaktivität aufweisen. Im Rahmen dieser Erfindung ist die Pflanzenzelle mit einem Glutathion-S-Transferase-Gen transformiert, welches im Zuge seiner Expression oder Ueberexpression der Pflanze eine Herbizid-Toleranz verleiht.

Die vorliegende Erfindung betrifft darüberhinaus auch aus transformierten Pflanzenzellen regenerierte Pflanzen sowie deren Samen, darüberhinaus auch die Nachkommen von aus transgenen Pflanzenzellen regenerierten Pflanzen sowie Mutanten und Varianten davon.

Die Erfindung bezieht sich ebenso auf chimäre genetische Konstruktionen, die ein Glutathion-S-Transferase-Gen enthalten, auf Klonierungsvehikel und Wirtsorganismen sowie Methoden zur Uebertragung der Herbizid-Toleranz auf Pflanzen.

Nachfolgend sollen die beigefügten Abbildungen kurz charakterisiert und erläutert werden:

Abbildung 1 legt die Sequenzierungs-Strategie für das pGTR200-cDNA-Insert von $Y_b200$ dar, einem Glutathion-S-Transferase-Gen aus der Rattenleber.

Abbildung 2 zeigt das Plasmid pCIB710, ein E.coli-Replikon, das den Promotor für das CaMV-35S-DNA-Transkript sowie dessen Terminator, und polyA-Zusatzsignal umfasst.

Abbildung 3 zeigt die Konstruktion des Plasmids pCIB12, ein E.coli-Replikon, enthaltend das chimäre Gen mit dem CaMV-35S-Promotor, das mit dem Glutathion-S-Transferase-cDNA-Gen aus Ratten-leber $Y_b200$, und dem CaMV Terminator verknüpft ist.

Abbildung 4A veranschaulicht die Konstruktion des Plasmids pCIB14, eines Replikon mit weitem Wirtsbereich, das zwei chimäre Gene innerhalb einer T-DNA Grenzsequenz enthält, wobei das chimäre Gen aus dem CaMV-35S-Promotor besteht, der mit dem Glutathion-S-Transferase-Gen $Y_b200$, dem CaMV-Terminator und dem Kanamycin-Resistenz-Gen, nos-neo-nos, verknüpft ist.

Abbildung 4B zeigt die Fertigstellung eines Teilklones mit Hilfe der in vivo Rekombinationsmethode.

Abbildung 4C zeigt die Fertigstellung eines Teilklones mit Hilfe der in vitro Verknüpfungsmethode.

Abbildung 5 zeigt Fluoreszenz-Induktionsmuster, die für nichttransgene Tabak-Blätter typisch sind. Die obere Kurve erhält man, nachdem die Blätter $10^{-5}$M Atrazin über einen Zeitraum von 48 Stunden aufgenommen haben. Die untere Kurve erhält man bei Aufnahme reiner Pufferlösung.

Abbildung 6 zeigt die Fluoreszenz-Induktionsmuster, die man in Blättern aus transgenen pCIB14 Tabkpflanzen nach 48-stündigem Aufsaugen von $10^{-5}$M Atrazin erhält, dabei lassen sich drei Klassen von Antworten ermitteln: (i) Keine Detoxifikation des Atrazin, obere Kurve; (ii) signifikante Detoxifikation des Atrazin, untere Kurve; (iii) eine mittlere Detoxifikation, mittlere Kurve.

Abbildung 7a zeigt die Konstruktion des Plasmids pCIB5.

Abbildung 7b zeigt die Konstruktion des Plasmids pCIB4.

Abbildung 7c zeigt die Konstruktion des Plasmids pCIB2.

Abbildung 7d/e beschreibt die Konstruktion des Plasmids pCIB10.

Abbildung 7f/g beschreibt die Konstruktion des Plasmids pCIB10a.

In der folgenden Beschreibung werden eine Reihe von Ausdrücken verwendet, die in der rekombinanten DNA Technologie, sowie in der Pflanzengenetik gebräuchlich sind.

Um ein klares und einheitliches Verständnis der Beschreibung und der Ansprüche sowie des Umfangs, der den besagten Ausdrücken zukommen soll, zu gewährleisten, werden die folgenden Definitionen aufgestellt:

Heterologe(s) Gen(e) oder DNA: Eine DNA Sequenz, die für ein spezifisches Produkt, Produkte oder eine biologische Funktion kodiert und die von einer anderen Spezies stammt als derjenigen, in welche das besagte Gen eingeschleusst wird; besagte DNA Sequenz wird auch als Fremdgen oder Fremd-DNA bezeichnet.

Homologe(s) Gen(e) oder DNA: Eine DNA Sequenz, die für ein spezifisches Produkt, Produkte oder eine biologische Funktion kodiert und die aus der gleichen Spezies stammt, in welche das besagte Gen eingeschleusst wird.

Pflanzen-Promotor: Eine Kontrollsequenz der DNA Expression, die die Transkription jeder beliebigen homologen oder heterologen DNA Gensequenz in einer Pflanze gewährleistet, sofern diese in operabler Weise mit einem solchen Promotor verknüpft ist.

Ueberproduzierender Pflanzen-Promotor (OPP): Pflanzen-Promotor, der in der Lage ist, in einer transgenen Pflanzenzelle die Expression einer jeden in operabler Weise verknüpften funktionalen Gensequenz(en) in einem Ausmass (gemessen in Form der RNA oder der Polypeptidmenge) zu bewirken, das deutlich höher liegt, als dies natürlicherweise in Wirtszellen, die nicht mit besagtem OPP transformiert sind, beobachtet wird.

Glutathion-S-Transferase: Die Definition dieses Enzyms erfolgt auf funktionale Art und Weise und schliesst jede Glutathion-S-Transferase (GST) mit ein, die in der Lage ist, in einer gegebenen und gewünschten Pflanze die Konjugation von Glutathion und einer elektrophilen Verbindung zu katalysieren. Dieser Ausdruck umfasst daher nicht nur das Enzym aus der spezifischen Pflanzenspezies, die in die genetische Transformation einbezogen ist, sondern schliesst auch Glutathion-S-Transferasen (GSTen) aus anderen Pflanzenspezies sowie aus Mikroorganismen oder Säugerzellen mit ein, sofern diese GST in der Lage sind, in der transgenen Pflanzenzelle ihre natürliche Funktion zu erfüllen.

Der Begriff GST schliesst Aminosäuresequenzen mit ein, die kürzer oder länger sind als die Sequenzen natürlicher GSTen, wie z.B. funktionelle Hybride oder Teilfragmente von GSTen oder deren Analoge.

Pflanze: Jedes photosynthetisch aktive Mitglied aus dem Reich Planta, das durch einen membranumhüllten Nukleus, ein in Form von Chromosomen organisiertes genetisches Material, membranumhüllte cytoplasmatische Organelle und der Fähigkeit zur Durchführung einer Meiose charakterisiert ist.

Pflanzen-Zelle: Strukturelle und physiologische Einheit der Pflanze, bestehend aus einem Protoplasten und einer Zellwand.

Pflanzengewebe: Eine Gruppe von Pflanzenzellen, die in Form einer strukturellen und funktionellen Einheit organisiert sind.

Pflanzenorgan: Ein abgegrenzter und deutlich sichtbar differenzierter Teil einer Pflanze, wie beispielsweise Wurzel, Stamm, Blatt oder Embryo.

## Detaillierte Beschreibung der vorliegenden Erfindung

Herbizidtolerante Pflanzen mit erhöhter GST-Enzymaktivität:
Die vorliegende Erfindung betrifft rekombinante DNA Moleküle, die
einer Pflanze eine, auf der Detoxifikation des Herbizids basierende
Herbizid-Toleranz verleihen.

Zu den bekannten Detoxifikationsmechanismen gehören die Hydroxylierung und Kohlenhydrat-Konjugation von Sulfonylharnstoff
[Hutchison et al., Pesticide Biochem. and Physiol., 22: 243-249
(1984)], die D-Aminosäure-Konjugation an 2-4-D [Davidonis et al.,
Plant Phys., 70: 357-360 (1982)] und die Konjugation von Glutathion
an elektrophile Verbindungen, katalysiert durch die Glutathion
S-Transferase.

Die vorliegende Erfindung betrifft insbesondere herbizidtolerante
Pflanzen, die mit einem rekombinanten DNA-Molekül transformiert
sind, das für ein Enzym kodiert, welches für die Detoxifikation von
Herbiziden verantwortlich ist. Weiterhin betrifft die vorliegende
Erfindung in erster Linie rekombinante DNA-Moleküle, die durch eine
Gen-Sequenz gekennzeichnet sind, welche für ein Glutathion-S-Transferase Polypeptid kodiert, sowie herbizidtolerante transformierte
Pflanzen, die eine erhöhte Glutathion S-Transferase Enzymaktivität
aufweisen. Die Glutathion enthaltenden Pflanzenzellen und Pflanzen
sind durch ein Glutathion S-Transferase Gen transformiert, das bei
der Expression in besagter Pflanzenzelle oder Pflanze die GST-
Enzymaktivität steigert und somit der Pflanze eine Toleranz gegenüber Herbiziden verleiht. Im Rahmen dieser Erfindung werden für die
Modifikation dieser Pflanzen genmanipulatorische Techniken verwendet.

Unter einer "herbizidtoleranten Pflanze" ist im Rahmen der vorliegenden Erfindung definitionsgemäss eine Pflanze zu verstehen, die in
Gegenwart einer normalerweise wirksamen Herbizidkonzentration
überlebt und vorzugsweise ein normales Wachstum zeigt.

Herbizid-Toleranz in Pflanzen bezieht sich erfindungsgemäss auf einen Detoxifikationsmechanismus in der Pflanze, auch wenn der Herbizid-Bindungs- oder -Zielort weiterhin sensitiv ist.

Unter Resistenz ist die maximal erreichbare Toleranz zu verstehen.

Die Detoxifikation muss von anderen Mechanismen zur Uebertragung einer Herbizid-Toleranz unterschieden werden, bei denen der Herbizid-Bindungs- oder -Zielort verändert wird und nicht länger sensitiv ist.

Im Rahmen der vorliegenden Erfindung bleibt der Herbizid-Bindungsort in der Pflanze unverändert sensitiv, wobei aber das Herbizid nicht gebunden wird, da es z.B. durch das GST-Enzym zuvor entgiftet wird.

Der im Rahmen der vorliegenden Erfindung verwendete Ausdruck "Herbizid-Toleranz" soll daher sowohl Toleranz als auch Resistenz gegenüber Herbiziden umfassen, wobei besagte Toleranz bzw. Resistenz auf die Detoxifikation von Herbiziden, beispielsweise aufgrund einer erhöhten GST-Enzymaktivität zurückzuführen ist. Eine herbizidtolerante Pflanze kann ohne Schädigungen in der Gegenwart bestimmter Herbizide überleben, die für herbizidsensitive Pflanzen letal sind oder deren Wachstum oder Vitalität beeinträchtigen.

Unter Herbiziden sind im Rahmen der vorliegenden Erfindung alle Herbizide zu verstehen, die detoxifiziert werden können, beispielsweise durch Bildung von Konjugate an pflanzliches Glutathion oder Glutathionanaloge oder -homologe, bzw. an jede elektrophile Verbindung. Besonders bevorzugt im Rahmen der vorliegenden Erfindung sind Herbizide, die einen Chlor-Rest aufweisen. Beispiele derartiger Herbizide, die aber keine Limitierung darstellen, sind im Rahmen der vorliegenden Erfindung: Triazine, einschliesslich der Chloratrazine, Acetamide, einschliesslich der Chloracetanilide, Sulfonylharnstoffe, Imidazolinone, Thiocarbamate, chlorierte Nitrobenzole, Diphenylether und andere. Einige spezifischen Beispiele solcher Herbizide umfassen das Atrazin, Alachlor, S-Ethyldipropylthiocarbamat und Diphenylether.
Siehe ebenso: Herbicide Resistance in Plants (H. LeBaron and
J. Gressel, ed., 1982).

Bei einigen der genannten Herbizide handelt es sich um potente
Photosynthesehemmer in der Pflanze.

Frear et al., Phytochemistry, 9: 2123-2132 (1970) (Chlortriazine);
Frear et al., Pesticide Biochem. and Physiol., 20: 299-310 (1983)
(Diphenylether); Lay et al., Pesticide Biochem. and Physiol., 6:
442-456 (1976) (Thiocarbamate); and Frear et al., Pesticide Biochem.
and Physiol., 23: 56-65 (1985) (Metribuzin). Andere Herbizide führen
zur Inaktivierung von Enzymen, die in der Biosynthese von Aminosäuren eine Rolle spielen.

Auch wenn der Ausdruck "Herbizide" verwendet wird um diese Verbindungen zu beschreiben, so ist der Gebrauch dieses Ausdruckes im
Rahmen dieser Erfindung nicht als limitierend zu betrachten. So gibt
es beispielsweise zahlreiche Insektizide und Pestizide (zur Kontrolle von Krankheiten, Parasiten und Frassfeinden), die bei Applikation auf die Pflanze schädliche Auswirkungen auf die Vitalität der
Pflanze haben. Das insektizid- oder pestizid-wirksame Agens kann
möglicherweise durch die Blätter, den Stamm oder aus dem Boden über
das Wurzelsystem in die Pflanze aufgenommen werden.

Darüberhinaus gibt es zahlreiche Xenobiotika, die zu den elektrophilen Verbindungen gehören und die in einer durch die GST-Enzymaktivität katalysierten Reaktion an Glutathion konjugiert werden
können. Diese xenobiotischen Verbindungen sind ebenso von der
vorliegenden Erfindung umfasst.

Eine spezifische Ausführungsform der vorliegenden Erfindung umfasst
Herbizide, die Sensibilisatoren darstellen, d.h. Inhibitoren der
GST-Enzymaktivität. Diese Sensibilisatoren hemmen den endogenen
Detoxifikationsmechanismus, indem sie ein GST-Sensibilisator-
Konjugat bilden.

Die gleichzeitige Applikation eines Herbizids und eines Sensibilisators, z.B. Tridiphan, auf eine Pflanze, führt zu einer Hemmung der enzymkatalysierten Konjugation zwischen Glutathion und dem Herbizid. Ezra et al., "Tridiphane as a Synergist for Herbicides in Corn (Zea mays) and Proso Millet (Panicum miliaceum), Weed Science, 33: 287-290 (1985).

Im Rahmen der vorliegenden Erfindung führt daher die Anwendung genmanipulatorischer Techniken zur Uebertragung einer GST-Enzymaktivität oder einer erhöhten GST-Enzymaktivität auf eine Pflanze, zu einer transgenen Pflanze, die eine erhöhte Toleranz bzw. Resistenz gegenüber Sensibilisatoren wie Tridiphan aufweist.

Auf diese Weise können beispielsweise ein Sensibilisator und ein Herbizid in Kombination auf Herbizid-sensitive und gleichzeitig auf Herbizid-tolerante Pflanzen gemäss der vorliegenden Erfindung appliziert werden. Diese Kombination von Sensibilisator und Herbizid wird in der Regel für die Herbizid-sensitiven Pflanzen toxisch sein, nicht aber für transgene Pflanzen.

Jede Pflanze, die Glutathion oder eine analoge Verbindung, wie beispielsweise Homo-Glutathion, enthält und die einer genetischen Manipulation unter Anwendung genmanipulatorischer Techniken zugänglich ist, kann im Rahmen dieser Erfindung verwendet werden. Die transgene Pflanze muss darüberhinaus in der Lage sein, das GST-Gen zu exprimieren.

Unter Pflanzen sollen im Rahmen der vorliegenden Erfindung Pflanzenzellen, Pflanzenprotoplasten, pflanzliche Gewebekulturen, die kultiviert und zur Bildung ganzer Pflanzen induziert werden können, aber auch Pflanzenkalli, Pflanzenklumpen sowie Pflanzenzellen als intakte Bestandteile einer Pflanze und Pflanzenteile verstanden werden.

Der Ausdruck "Pflanze" bezieht sich auch auf Pollen, der mit Hilfe genmanipulatorischer Techniken transformierbar ist.

Die höchsten Glutathionkonzentrationen in Pflanzen findet man in der Regel in den subzellulären Kompartimenten des pflanzlichen Gewebes, wie z.B. in den pflanzlichen Plastiden, insbesondere in den Chloroplasten [Rennenberg, H., Phytochemistry, 21: 2771-2781 (1982)]. Glutathion hat eine Gamma-L-Glutamyl-L-Cysteinyl-Glycin-Struktur. Eine homologe Form des Glutathion, das Homo-Glutathion, wurde in einigen Pflanzen gefunden. Es hat die Struktur des Gamma-L-Glutamyl-L-Cysteinyl-beta-Alanins [Carnegie, P., Biochem. J., 89: 459-471 (1963) und Carnegie, P., Biochem. J., 89: 471-478 (1963)].

Pflanzen weisen unterschiedliche Mengen an Glutathion oder Homo-Glutathion auf. So findet man beispielsweise in verschiedenen Leguminosen in der Hauptsache Homo-Glutathion, während andere Leguminosen vorzugsweise Glutathion enthalten. Normalerweise liegt in Fällen, in denen eine der beiden Komponenten, entweder Homo-Glutathion oder Glutathion, in der Pflanze vorherrscht, die andere Komponente nur in geringer Konzentration, vor. (Rennenberg, supra).

Die für das Glutathion-S-Transferase (GST)-Gen kodierende Region, die gemäss der vorliegenden Erfindung verwendet wird, kann homologen oder heterologen Ursprungs sein, im Verhältnis zu der Pflanzenzelle oder der Pflanze, die transformiert werden soll. Es ist jedoch auf alle Fälle notwendig, dass die Gensequenz, die für GST kodiert exprimiert wird und zur Produktion eines funktionstüchtigen Enzyms oder Polypeptids in der resultierenden Pflanzenzelle führt. Bestandteil der vorliegenden Erfindung sind daher Pflanzen, die entweder homologe oder heterologe GST-Gene besitzen und die das GST-Enzym exprimieren.

Weiterhin kann die GST aus anderen Pflanzen-Spezies oder aus Organismen stammen, die einer anderen taxonomischen Einheit angehören, so z.B. aus Mikroben oder aus Säugern.

Wie zuvor beschrieben, handelt es sich bei dem GST-Enzym um eine Klasse multifunktioneller Enzyme. Daher ist es notwendig, ein GST-Gen auszuwählen, das die Konjugation von Glutathion und einer elektrophilen Verbindung katalysiert.

Da Glutathion als Substrat der GST fungiert, war es vor dieser Erfindung ungewiss, ob das GST-Enzym, das für die Konjugation von Glutathion spezifisch ist, Homo-Glutathion in transformierten Pflanzen akzeptiert. Frear et al., Phytochemistry, 9: 2123-2132 (1970) (weist nach, dass Glutathion-S-Transferase spezifisch für reduziertes Glutathion ist). Das benötigte GST-Enzym, das spezifisch ist für Glutathion, kann durch Verwendung eines Assays, bei dem zur Differenzierung der verschiedenen Glutathion-S-Transferasen die Substratspezifität bestimmt wird, identifiziert und ausgewählt werden. In einem typischen Assay kann die Glutathion-spezifische GST mit Hilfe einer Affinitätschromatographie charakterisiert werden [Tu et al., Biochem. and Biophys. Research Comm., 108: 461-467 (1982); Tu et al., J. Biol. Chem., 258: 4659-4662 (1983); and Jakoby et al., in Glutathione; Metabolism and Function, (Raven Press, New York, 1976)].

In einer spezifischen Ausführungsform dieser Erfindung handelt es sich bei der GST um eine pflanzliche GST, die der zu transformierenden Pflanze homolog ist. In einer weiteren Ausführungsform dieser Erfindung, handelt es sich bei der GST um eine pflanzliche GST, die heterolog ist zu der zu transformierenden Pflanze.

Zu den Pflanzen, die einen GST-Ueberschuss haben, gehören Mais und Sorghum. Eine weitere Ausführungsform der vorliegenden Erfindung beinhaltet eine Säuger-GST. Säuger-GSTen sind bekannt und beschrieben bei Reddy et al., Archives of Biochem. and Biophys., 224: 87-101 (1983) (Schafsleber); Tu et al., J. Biol. Chem., 258: 4659-4662 (1983) (Rattengewebe aus Herz, Niere, Leber, Lunge, Milz und Testis). Das bevorzugte GST-Gen ist gekennzeichnet durch die kodierende Region des GST-Gens aus Rattenleber, insbesondere durch

$Y_b$200, beschrieben in Beispiel I sowie das vervollständigte $Y_b$187 beschrieben in Beispiel IB. Ein weiterer Bestandteil der vorliegenden Erfindung betrifft die codierende Region eines GST-Gens aus dem Rattengehirn, insbesondere den cDNA Klon G1$Y_b$, der in Beispiel IE beschrieben ist. Es sind jedoch auch andere Gene bekannt, die im Rahmen der vorliegenden Erfindung verwendet werden können. Siehe: Mannervik, B., Adv. Enzymol. Relat. Areas Mol. Biol., 57: 357-417 (1985), per Referenz in diese Erfindung inkorporiert.

Die für die Glutathion-S-Transferase kodierende DNA-Sequenz kann ausschliesslich aus genomischer bzw. aus cDNA konstruiert sein. Eine andere Möglichkeit besteht in der Konstruktion einer hybriden DNA-Sequenz bestehend sowohl aus cDNA als auch aus genomischer DNA. In diesem Fall kann die cDNA aus demselben Gen stammen wie die genomische DNA oder aber, sowohl die cDNA, wie auch die genomische DNA können aus verschiedenen Genen stammen. In jedem Fall können aber, sowohl die genomischen DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehrteren Organismen, die mehr als einem Stamm, einer Varietät oder einer Spezies derselben Gattung angehören, oder aber von Organismen, die mehr als einer Gattung derselben oder einer anderen taxonomischen Einheit (Reich) angehören, abstammen.

Die verschiedenen DNA-Sequenzabschnitte können mit Hilfe an sich bekannter Methoden miteinander zu einer vollständigen Glutathion-S-Transferase kodierenden DNA-Sequenz verknüpft sein. Geeignete Methoden schliessen beispielsweise die in vivo Rekombination von DNA-Sequenzen, die homologe Abschnitte aufweisen sowie die in vitro Verknüpfung von Restriktionsfragmenten mit ein.

Es werden somit eine Vielzahl von Ausführungsformen von dem breiten Konzept dieser Erfindung umfasst.

Eine dieser erfindungsgemässen Ausführungsformen ist durch eine chimäre Gen-Sequenz gekennzeichnet, enthaltend:

(a) eine erste Gen-Sequenz, die für ein Glutathion-S-Transferase Polypeptid kodiert, das nach erfolgter Expression des Gens in einer gegebenen Pflanzenzelle Glutathion S-Transferase-Aktivität aufweist; und

(b) eine oder mehrere zusätzliche Gensequenzen, die in operabler Weise mit beiden Seiten der für GST-kodierenden Region verknüpft sind. Diese zusätzlichen Gensequenzen enthalten Promotor- und/oder Terminator-Regionen. Die pflanzlichen regulatorischen Sequenzen können im Verhältnis zu der Wirtszelle heterolog oder homolog sein .

Jeder Promotor und jeder Terminator, der in der Lage ist, eine Induktion der Expression einer für GST-kodierenden Region zu bewirken, kann als Bestandteil der chimären Gensequenz verwendet werden. Beispiele geeigneter Promotoren und Terminatoren sind z.B. solche der Nopalin-Synthase-Gene (nos), der Octopin-Synthase-Gene (ocs) sowie der Cauliflower Mosaik Virus Gene (CaMV).

Ein wirksamer Vertreter eines Pflanzenpromotors, der Verwendung finden kann, ist ein überproduzierender Pflanzenpromotor. Diese Art von Promotoren sollte, sofern sie in operabler Weise mit der Gensequenz für die GST verknüpft ist, in der Lage sein, die Expression besagter GST in einer Weise zu vermitteln, dass die transformierte Pflanze gegenüber einem Herbizid aufgrund der vorhandenen bzw. aufgrund einer erhöhten GST-Enzymaktivität tolerant ist.

Ueberproduzierende Pflanzenpromotoren, die im Rahmen der vorliegenden Erfindung zur Anwendung kommen können, schliessen den Promotor der kleinen Untereinheit (small subunit; ss) der Ribulose-1,5-bisphosphat-Carboxylase aus Sojabohnen [Berry-Lowe et al., J. Molecular and App. Gen., 1: 483-498 (1982)] sowie den Promotor des Chlorophyll-a/b-Bindungsproteins ein. Diese beiden Promotoren sind dafür bekannt, dass sie in eukaryontischen Pflanzenzellen durch Licht induziert werden [siehe z.B. Genetic Engineering of Plants, an Agricultural Perspective, A. Cashmore, Plenum, New York 1983, Seite 29-38; Coruzzi G. et al., The Journal Of Biological Chemistry, 258: 1399 (1983) und Dunsmuir, P. et al., Journal of Molecular and Applied Genetics, 2: 285 (1983)].

Die chimäre Gensequenz, die aus einem Glutathion-S-Transferase-Gen besteht, das in operabler Weise mit einem pflanzlichen Promotor verknüpft ist, kann in einen geeigneten Klonierungsvektor eingespleisst werden. Im allgemeinen verwendet man Plasmid- oder Virus-(Bak-teriophage)-Vektoren mit Replikations- und Kontrollsequenzen, die von Spezies stammen, die mit der Wirtszelle kompatibel sind.

Der Klonierungsvektor trägt in der Regel einen Replikationsursprung, ausserdem spezifische Gene, die zu phenotypischen Selektionsmerk-malen in der transformierten Wirtszelle führen, insbesondere zu Resistenzen gegenüber Antibiotika oder gegenüber bestimmten Herbiziden. Die transformierten Vektoren können anhand dieser phenotypischen Marker nach einer Transformation in einer Wirtszelle selektiert werden.

Als Wirtszellen kommen im Rahmen dieser Erfindung Prokaryonten in Frage, einschliesslich bakterieller Wirte, wie z.B. A.tumefaciens, E.coli, S.typhimurium und Serratia marcescens, sowie Cyanobakterien. Ferner können auch eukaryontische Wirte wie Hefen, mycelbildende Pilze und Pflanzenzellen im Rahmen dieser Erfindung verwendet werden.

Der Klonierungsvektor und die mit diesem Vektor transformierte Wirtszelle werden erfindungsgemäss dazu verwendet, die Kopienzahl des Vektors zu erhöhen. Mit einer erhöhten Kopienzahl ist es möglich, den Vektor, der das GST-Gen trägt, zu isolieren und z.B. für die Einschleusung der chimären Gensequenz in die pflanzliche Zelle zu verwenden.

Die Einschleusung von DNA in Wirtszellen kann mit Hilfe von an sich bekannten Verfahren durchgeführt werden. So können beispielsweise bakterielle Wirtszellen nach einer Behandlung mit Calciumchlorid transformiert werden. In Pflanzenzellen lässt sich DNA durch direkten Kontakt mit den aus den Zellen hergestellten Protoplasten einschleusen.

Eine andere Möglichkeit zur Einführung von DNA in Pflanzenzellen besteht darin, dass man die Zellen mit Viren oder mit _Agrobacterium_ in Kontakt bringt. Dies kann durch Infektion sensitiver Pflanzenzellen oder durch Co-Cultivierung von Protoplasten, die sich aus Pflanzenzellen ableiten, bewerkstelligt werden.

Diese Verfahren werden im folgenden noch genau diskutiert.

Für die direkte Einschleusung von DNA in Pflanzenzellen stehen eine Reihe von Verfahren zur Verfügung. So kann das genetische Material, das in einem Vektor enthalten ist, beispielsweise mit Hilfe von Mikropipetten für den mechanischen Transfer der rekombinanten DNA direkt in die Pflanzenzellen mikroinjiziert werden. Das genetische Material kann ebenso nach einer Behandlung der Protoplasten mit Polyethylenglykol in diese eingebracht werden. [Paszkowski _et al._, EMBO J., **3**: 2717-22 (1984)].

Ein weiterer Gegenstand der vorliegenden Erfindung betrifft die Einschleusung des GST-Gens in die Pflanzenzelle mit Hilfe der Elektroporation [Shillito _et al._, _Biotechnology_, **3**: 1099-1103 (1985); Fromm _et al._, _Proc. Natl. Acad. Sci. USA_, **82**: 5824 (1985)].

Bei dieser Technik werden pflanzliche Protoplasten in der Gegenwart von Plasmiden, die das GST-Gen enthalten, einer Elektroporation unterzogen.

Elektrische Impulse hoher Feldstärke führen zu einer reversiblen Permeabilitätserhöhung von Biomembranen und ermöglichen damit die Einschleusung der Plasmide. Elektroporierte pflanzliche Protoplasten erneuern ihre Zellwand, teilen sich und bilden Kallusgewebe. Die Selektion der transformierten Pflanzenzellen mit dem exprimierten GST-Enzym kann mit Hilfe der zuvor beschriebenen phenotypischen Marker erfolgen.

Das Cauliflower Mosaik Virus (CaMV) kann im Rahmen dieser Erfindung ebenso als Vektor für die Einschleusung des GST-Gens in die Pflanze verwendet werden (Hohn et al., in "Molecular Biology of Plant Tumors", Academic Press, New York, 1982, Seite 549-560; Howell, US-Patent Patent No. 4,407,956).

Das gesamte virale DNA-Genom von CaMV wird dabei in ein bakterielles Elternplasmid integriert unter Ausbildung eines rekombinanten DNA Moleküls, das in Bakterien vermehrt werden kann. Nach erfolgter Klonierung wird das rekombinante Plasmid mit Hilfe von Restriktionsenzymen entweder zufällig oder an ganz spezifischen nicht essentiellen Stellen innerhalb des viralen Teils des rekombinanten Plasmids gespalten, z.B. innerhalb des Gens, das für die Uebertragbarkeit des Virus durch Blattläuse kodiert, um die GST-Gensequenz einbauen zu können.

Ein kleines Oligonucleotid, ein sog. Linker, der eine einzige, spezifische Restriktionserkennungsstelle besitzt, kann ebenfalls integriert werden. Das so modifizierte rekombinante Plasmid wird erneut kloniert und durch Einspleissen der GST-Gensequenz in eine nur einmal vorkommende Restriktionsstelle weiter modifiziert.

Der modifizierte virale Anteil des rekombinanten Plasmids wird dann aus dem bakteriellen Eltern-Plasmid ausgeschnitten und für die Inokulation der Pflanzenzellen oder der gesamten Pflanzen verwendet.

Eine andere Methode zur Einschleusung der GST-Gene in die Zelle bedient sich der Infektion der Pflanzenzelle mit Agrobacterium tumefaciens, welches mit dem GST-Gen transformiert ist. Die transgenen Pflanzenzellen werden anschliessend unter geeigneten, dem Fachmann bekannten Kultivierungsbedingungen kultiviert, so dass sie
Schösslinge und Wurzeln ausbilden und letztlich ganze Pflanzen
resultieren.

Die GST-Gensequenzen lassen sich beispielsweise mit Hilfe des
Ti-Plasmids von Agrobacterium tumefaciens in geeignete Pflanzenzellen überführen [DeCleene et al., Bot. Rev., 47: 147-194 (1981);
Bot. Rev., 42: 389-466 (1976)]. Das Ti-Plasmid wird im Verlaufe der
Infektion durch Agrobacterium tumefaciens auf die Pflanze übertragen
und stabil uns Pflanzengenom integriert. [Horsch et al., Science,
233: 496-498 (1984); Fraley et al., Proc. Natl. Acad. Sci. USA, 80:
4803 (1983)].

Für Pflanzen, deren Zellen für eine Infektion mit Agrobacterium
nicht sensitiv sind, kann man auf die Co-Kultivierung von Agrobacterium mit dem entsprechenden Protoplasten zurückgreifen.

Ti-Plasmide besitzen zwei Regionen, die für die Herstellung transformierter Zellen essentiell sind. Eine davon, die Transfer-DNA
Region, wird auf die Pflanze übertragen und führt zur Induktion von
Tumoren. Die andere, die virulenzverleihende (vir) Region, ist nur
für die Ausbildung, nicht aber für die Aufrechterhaltung der Tumoren
essentiell. Die Transfer-DNA Region kann in ihren Dimensionen durch
Einbau der GST-Gensequenz vergrössert werden, ohne dass die Uebertragungsfähigkeit beeinträchtigt wird. Durch Entfernen der tumorverursachenden Gene, wodurch die transgenen Pflanzenzellen nichttumorös bleiben und durch Einbau eines selektionierbaren Markers,
kann das modifizierte Ti-Plasmid als Vektor für den Transfer der
erfindungsgemässen Genkonstruktion in eine geeignete Pflanzenzelle
verwendet werden.

Die vir-Region bewirkt den Transfer der T-DNA Region von Agrobacterium auf das Genom der Pflanzenzelle, unabhängig davon, ob die
T-DNA-Region und die vir-Region auf demselben Vektor oder auf verschiedenen Vektoren innerhalb derselben <u>Agrobacterium</u>-Zelle vorliegen. Eine vir-Region auf einem Chromosom induziert ebenso den Transfer der T-DNA von einem Vektor in eine Pflanzenzelle.

Bevorzugt wird ein System zur Uebertragung einer T-DNA-Region von einem <u>Agrobacterium</u> in Pflanzenzellen, das dadurch gekennzeichnet ist, dass die vir-Region und die T-DNA-Region auf verschiedenen Vektoren liegen. Ein solches System ist unter dem Begriff "binäres Vektor-System" bekannt und der die T-DNA enthaltende Vektor wird als ein "binärer Vektor" bezeichnet.

Jeder T-DNA enthaltende Vektor, der in Pflanzenzellen übertragbar ist und der eine Selektion der transformierten Zellen erlaubt, ist für die Verwendung im Rahmen dieser Erfindung geeignet. Bevorzugt ist ein Vektor, der aus einem Promotor, einer kodierenden Sequenz und pCIB10 hergestellt ist.

Jeder Vektor mit einer vir-Region, der den Transfer einer T-DNA-Region von <u>Agrobacterium</u> auf Pflanzenzellen bewirkt, kann im Rahmen dieser Erfindung verwendet werden. Der bevorzugte Vektor mit einer vir-Region ist pCIB542.

Pflanzenzellen oder Pflanzen, die gemäss der vorliegenden Erfindung transformiert worden sind, können mit Hilfe eines geeigneten phenotypischen Markers, der neben dem GST-Gen Bestandteil der DNA ist, selektioniert werden. Beispiele solcher phenotypischer Marker, die aber nicht als limitierend aufzufassen sind, beinhalten Antibiotikaresistenz-Marker, wie beispielsweise Kanamycinresistenz- und Hygromycinresistenz-Gene, oder Herbizidresistenz-Marker. Andere phenotypische Marker sind dem Fachmann bekannt und können ebenfalls im Rahmen dieser Erfindung verwendet werden.

Alle Pflanzen, deren Zellen sich durch direkte Einschleusung von DNA oder durch Kontakt mit <u>Agrobacterium</u> transformieren lassen und anschliessend zu vollständigen Pflanzen regenerierbar sind, können dem erfindungsgemässen Verfahren zur Herstellung transgener ganzer Pflanzen, die das übertragene GST-Gen enthalten, unterzogen werden. Es existiert eine ständig wachsende Anzahl von Hinweisen, die darauf schliessen lassen, dass sich im Prinzip alle Pflanzen aus kultivierten Zellen oder Geweben regenieren lassen, einschliesslich, aber nicht beschränkt auf, alle wichtige Getreidearten, Zuckerrohr, Zuckerrüben, Baumwolle, Obstbäumen und anderer Baumarten, Leguminosen sowie Gemüsen.

Zu den Zielkulturen im Rahmen der vorliegenden Erfindung zählen beispielsweise auch jene, ausgewählt aus der Reihe: <u>Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum und Sorghum</u>, einschliesslich derjenigen, ausgewählt aus der Reihe: <u>Ipomoea, Passiflora, Cyclamen, Malus, Prunus, Rosa, Rubus, Populus, Santalum, Allium, Lilium, Narcissus, Ananas, Arachis, Phaseolus und Pisum</u>.

Die Kenntnisse darüber, inwieweit alle diese Pflanzen mit Hilfe von <u>Agrobacterium</u> transformiert werden können, sind zur Zeit noch beschränkt. <u>In vitro</u> lassen sich beispielsweise auch Pflanzenspezies transformieren, die keine natürlichen Wirtspflanzen für <u>Agrobacterium</u> sind. So sind beispielsweise monokotyle Pflanzen, insbesondere die Getreidearten und Gräser, keine natürlichen Wirte für <u>Agrobacterium</u>.

Es gibt in der Zwischenzeit vermehrt Hinweise darauf, dass sich auch Monokotyledonen mit <u>Agrobacterium</u> transformieren lassen, so dass unter Verwendung von neuen experimentellen Ansätzen, die jetzt verfügbar werden, auch Getreide und Gräser-Spezies einer Transformation zugänglich sind [Grimsley, N., <u>et al.</u>, <u>Nature</u>, <u>325</u>: 177-179 (1987)].

Die Regeneration von in Kultur gehaltenen Protoplasten zu ganzen Pflanzen ist bei Evans, et al., "Protoplast Isolation and Culture", in Handbook of Plant Cell Culture, 1: 124-176 (MacMillan Publishing Co. New York 1983); M.R. Davey, "Recent Developments in the Culture and Regenration of Plant Protoplasts", Protoplasts, 1983 - Lecture Proceedings, Seite 19-29, (Birkhäuser, Basel 1983); P.J. Dale, "Protoplast Culture and Plant Regeneration of Cereals and Other Recalcitrant Crops", in Protoplasts 1983 - Lecture Proceedings, Seite 31-41, (Birkhäuser, Basel 1983); und H. Binding, "Regeneration of Plants", in Plant Protoplasts, Seite 21-37, (CRC Press, Boca Raton 1985) beschrieben.

Die Regeneration unterscheidet sich von Pflanzenspezies zu Pflanzenspezies. Im allgemeinen aber wird zunächst eine Suspension von transformierten Protoplasten, Zellen oder von Gewebe, die zahlreiche Kopien der GST-Gene enthalten, hergestellt. Ausgehend von diesen Suspensionen kann anschliessend die Induktion der Embryobildung erfolgen. Man lässt die Entwicklung der Embryonen bis zum Stadium der Reife und Keimung fortschreiten, wie dies auch bei natürlicherweise vorkommenden Embryonen der Fall ist. Die Kulturmedien enthalten in der Regel verschiedene Aminosäuren und Hormone, wie z.B. Auxin und Cytokinine. Es erweist sich ebenfalls als vorteilhaft, dem Medium Glutaminsäure und Prolin zuzugeben, insbesondere bei Spezies wie Mais und Alfalfa. Die Spross- und Wurzelbildung erfolgt im allgemeinen simultan. Eine effiziente Regeneration hängt in erster Linie vom Medium, vom Genotyp und von der Vorgeschichte der Kultur ab. Werden diese drei Variablen hinreichend kontrolliert, dann ist die Regeneration vollständig reproduzier- und wiederholbar.

Pflanzen, die für die erfindungsgemässe Verwendung geeignet sind, schliessen beispielsweise Spezies aus den Gattungen Lotus, Medicago, Onobrychis, Trifolium, Trigonella, Citrus, Linum, Manihot, Daucus, Arabidopsis, Brassica, Raphanus, Sinapis, Atropa, Capsicum, Datura, Hyoscyamus, Lycopersicon, Nicotiana, Solanum, Petunia, Majorana, Cichorium, Helianthus, Lactuca, Asparagus, Antirrhinum, Panicum,

Pennisetum, Ranunculus, Salpiglossis, Glycine, Gossypium, Malus, Prunus, Rosa, Populus, Allium, Lilium, Narcissus, Ananas, Arachis, Phaseolus und Pisum ein.

Nachdem man herausgefunden hat, dass Oryza (Reis) aus Protoplasten zu ganzen Pflanzen regeneriert werden kann, sollte es jetzt auch möglich sein, andere Pflanzen aus der Familie der Gramineae zu regenerieren. Es können im Rahmen der vorliegenden Erfindung daher auch die folgenden Pflanzen verwendet werden: Lolium, Zea, Triticum, Sorghum und Bromus.

Besonders bevorzugt gemäss dieser Erfindung sind Pflanzen aus den Gattungen Nicotana spp. (z.B. Tabak), Glycine spp. (insbesondere Glycine max, Sojabohne) und Gossypium spp. (Baumwolle).

Die reifen Pflanzen, die aus transformierten Pflanzenzellen herangezogen wurden, werden zur Samenproduktion mit sich selbst gekreuzt. Einige der Samen enthalten die Gene für eine gesteigerte GST-Enzymaktivität in einem Verhältnis, das genau den etablierten Gesetzen der Vererbung gehorcht. Diese Samen können zur Produktion herbizidtoleranter Pflanzen verwendet werden. Die Toleranz dieser Samen lässt sich beispielsweise durch Wachstum der Samen in herbizidhaltiger Erde bestimmen. Alternativ dazu kann auch die Herbizid-Toleranz der transformierten Pflanze bestimmt werden, und zwar durch Applikation des Herbizids auf die Pflanze.

Homozygote Linien können durch wiederholte Selbstfertilisation und Herstellung von Inzuchtlinien gewonnen werden. Diese Inzuchtlinien können dann wiederum zur Entwicklung von herbizidtoleranten Hybriden verwendet werden. Bei diesem Verfahren wird eine herbizidtolerante Inzuchtlinie mit einer anderen Inzuchtlinie zur Produktion herbizidresistenter Hybride gekreuzt.

Teile, die von regenerierten Pflanzen erhalten werden können, wie z.B. Blüten, Samen, Blätter, Zweige, Früchte u.a. sind ebenfalls Bestandteil der vorliegenden Erfindung, sofern diese Teile herbizidtolerante Zellen beinhalten. Nachkommen (einschliesslich hybrider Nachkommen), Varietäten und Mutanten der regenerierten Pflanzen sind ebenfalls Bestandteil der vorliegenden Erfindung.

## Verwendung der GST-Genkonstruktionen und herbizidtoleranter Pflanzen

Die zuvor beschriebenen GST Genkonstruktionen können in Vektoren als Zwischenprodukte bei der Herstellung herbizidtoleranter Pflanzenzellen, Pflanzenorgane, Pflanzengewebe sowie ganzer Pflanzen verwendet werden.

Die Bedeutung herbizidtoleranter Pflanzen gemäss der vorliegenden Erfindung ist offensichtlich. Diese Pflanzen ermöglichen es den Landwirten nämlich, zunächst eine herbizidtolerante Feldfrucht auszupflanzen und das Feld anschliessend zur Bekämpfung von Unkräutern mit dem Herbizid zu behandeln, ohne dabei die Feldfrucht zu schädigen.

Darüberhinaus erlaubt es eine herbizidtolerante Pflanze den Landwirten, Feldfrüchte auf Flächen anzupflanzen, die zuvor mit Herbiziden behandelt worden sind, beispielsweise im Zuge einer Fruchtfolge, bei der einer von Natur aus toleranten Pflanze eine natürlicherweise sensitive Pflanze folgt. Diese herbizidbehandelten Anbauflächen können einen "Herbizidüberschuss" im Boden enthalten, so dass von Natur aus herbizidsensitive Pflanzen im Rahmen einer Fruchtfolge durch diesen Herbizidüberschuss im Boden geschädigt werden können, wenn sie sich nicht als tolerant erweisen [(Sheets, T., Residue Reviews, 32: 287-310 (1970); Burnside et al., Weed Science, 19: 290-293 (1971)].

Landwirte pflanzen beispielsweise in der Regel Mais und Sojabohnen in wechselnder Folge an. Während die Maispflanzen von Natur aus tolerant sind gegenüber bestimmten Herbiziden, wie z.B. verschiedenen Triazinen, wie Atrazin, können die empfindlicheren Sojabohnenpflanzen geschädigt werden, wenn sie auf eine Fläche ausgepflanzt
werden, die zuvor mit Herbiziden behandelt worden ist.
Bei der Verwendung einer herbizidtoleranten Pflanze gemäss vorliegender Erfindung, wird eine Schädigung aufgrund eines Herbizid-
überschusses im Boden vermieden [Fink et al., Weed Science, 17:
35-36 (1969) (Sojabohnen); Khan et al., Weed Research, 21: 9-12
(1981) (Hafer und Timotheuspflanzen); Brinkman et al., Crop Science,
20: 185-189 (1980) (Hafer); Eckert et al., J. Range Mgmt., 25:
219-224 (1972) (Quecke)].

Die vorliegende Erfindung schliesst ebenfalls ein Verfahren zur
Pflanzenkontrolle mit ein, welches sich dadurch kennzeichnet, dass
man eine Mischpopulation bestehend aus einer herbizidsensitiven
Pflanze, wie z.B. einem Unkraut, und einer erfindungsgemäss
herbizidtoleranten Pflanze mit einer für die Bekämpfung der
herbizidsensitiven Pflanze wirksamen Menge eines Herbizids in
Kontakt bringt. Daher ist das Verfahren der Blattbehandlung von
herbizidtoleranten Pflanzen und herbizidsensitiven Unkräutern mit
Herbiziden auf dem Feld, wobei beide Pflanzentypen im Verlaufe der
Behandlungsoperation gleichzeitig mit dem Herbizid in Kontakt
gebracht werden, ebenfalls ein Gegenstand der vorliegenden Erfindung.

Ein weiterer Gegenstand der vorliegenden Erfindung betrifft ein
zuvor bereits beschriebenes Verfahren der Pflanzenkontrolle, das
sich dadurch kennzeichnet, dass ein Herbizid und ein Sensibilisator
in einer Mischpopulation gleichzeitig sowohl auf herbizidtolerante
als auch auf herbizidsensitive Pflanzen appliziert werden.

Der Begriff "pflanzenkontrollierende Menge eines Herbizids" beschreibt auf funktionelle Weise eine Aufwandmenge eines Herbizids,
die in der Lage ist, das Wachstum oder die Entwicklung einer
bestimmten Pflanze zu beeinträchtigen. Die Aufwandmenge kann demnach
entweder möglichst klein gehalten werden, so dass sie für einen Rückgang bzw. eine Unterdrückung des Wachstums oder der Entwicklung ausreicht, oder aber sie kann so gross gewählt werden, dass eine irreversible Schädigung der sensitiven Pflanze eintritt.

Die tatsächliche Aufwandmenge hängt zum einem von dem verwendeten Herbizid, zum anderen von der zu kontrollierenden Pflanze ab. Für dikotyle Pflanzen und Unkräuter z.B. werden in der Regel Aufwandmengen zwischen 0,5 und 1,5 kg/ha benötigt. Für monokotyle Pflanzen liegen die benötigten Herbizidkonzentrationen im allgemeinen zwischen 0,5 und etwa 2,0 kg/ha. Verschiedene Herbizide, wie beispielsweise die Sulfonylharnstoffe, sind dafür bekannt, dass sie bereits in sehr geringen Aufwandmengen wirksam sind.

Die Herbizide können mit den entsprechenden Pflanzen unter Verwendung gut bekannter Sprüh- oder Streuverfahren in Kontakt gebracht werden. So kann beispielsweise die vorpublizierte Blattapplikation zur Kontrolle von Unkräutern durch Atrazin in Gegenwart von Atrazintoleranten Pflanzen gemäss vorliegender Erfindung verwendet werden.

Nach der allgemeinen Beschreibung der vorliegenden Erfindung soll nun zum besseren Verständnis auf spezifische Ausführungsbeispiele Bezug genommen werden, die zum Zwecke der Illustration in die Beschreibung mitaufgenommen werden, und die keinen limitierenden Charakter haben, es sei denn, es wird speziell darauf hingewiesen.

Beispiele

Die in den folgenden Beispielen verwendeten Verfahren sind generell bei Maniatis et al., Molecular Cloning, Cold Spring Harbor Laboratory, (1982), beschrieben. Enzyme können, wenn nicht extra darauf hingewiesen wird, von New England Biolabs bezogen werden. Sie werden entsprechend den Angaben des Herstellers verwendet, es sei denn, es wird gesondert darauf hingewiesen.

Beispiel IA: Isolierung des GST-cDNA-Klons $Y_b$ 200 Antikörper:

Antisera gegen homogene Leber-Glutathion-S-Transferasen (GSTen) (affinitätschromatographische Fraktion) werden entsprechend vorbeschriebener Verfahren hergestellt [Tu et al., Nucleic Acids Res., 10: 5407-5419 (1982)]. Die IgG Fraktion wird über eine Protein-A-Sepharose (Pharmacia) Säule gereinigt und durch Ultrafiltration (Amicon, XM-50 Membran) aufkonzentriert, entsprechend der Beschreibung bei Kraus und Rosenberg [Kraus & Rosenberg, Proc. Natl. Acad. Sci. USA, 79: 4015-4019 (1982)]. Sie wird anschliessend in 50 %igem Glycerol und 0,2 mg/ml Heparin bei -18°C aufbewahrt.


Isolierung von Polysomen

Die Lebern (ca. 26 g) von zwei männlichen Sprague-Dawley-Ratten (Körpergewicht ca. 300 g) werden mit einem Potter-Elvehjem-Homogenisator in 150 ml (Endvolumen) 50 mM Tris-HCl, pH 7,5, 25 mM MgCl₂, 0,25 M Sucroselösung enthaltend Bentonit (1 mg/ml), Heparin (0,2 mg/ml) und Cycloheximid (1 µg/ml) in verschiedenen Aliquots zu einem 15%igen (w/v) Homogenisat homogenisiert. Die Polysomenisolation wird exakt nach dem von Kraus und Rosenberg (siehe oben) beschriebenen Verfahren durchgeführt. Die Ausbeuten vor und nach der Dialyse betragen 1389 $A_{260}$ Einheiten bzw. 1208 $A_{260}$ Einheiten.


Immobilisierung der Polysomen-Antikörperkomplexe und Elution spezifischer mRNA

1130 $A_{260}$ Polysomen-Einheiten werden für die Immunabsorption mit Anti-GST IgG (7,1 mg) wiedergewonnen. Die Protein A-Sepharose Affinitätschromatographie sowie die Elution gebundener RNA-Moleküle werden entsprechend den Anweisungen bei Kraus und Rosenberg supra durchgeführt. Die eluierten RNA-Moleküle werden sofort an 0,5 M NaCl und 0,5 % Dodecylsulfat angepasst und über eine Oligo(dT)-Cellulose Säule weiter gereinigt [Aviv & Leder, Proc. Natl. Acad. Sci. USA, 69: 1408-1412 (1972); Bantle et al., Anal. Biochem., 72: 413-417 (1976)]. Die gereinigten Poly(A+)-RNA Moleküle werden anhand einer in vitro Translation und einer Immunpräzipitation [Tu et al., Nucleic Acids Res., 10: 5407-5419 (1982); Pelham and Jackson, Eur. J. Biochem., 67: 247-256 (1976)] analysiert, bevor sie für die
cDNA Synthese verwendet werden. Das immunpräzipitierte Material wird
auf einem Dodecylsulfat Polyacrylamid-Gel aufgetrennt und durch
Fluorographie sichbar gemacht [Laemmli, Nature, 227: 680-684 (1970);
Swanstrom and Shenk, Anal. Biochem., 86: 184-192 (1978)].

Isolierung der GST-cDNA Klone

Die Synthese der cDNA wird, abgesehen von kleineren Abweichungen,
analog zu der Methode von Okayama und Berg [Okayama and Berg,
Mol. Cell Biol., 2: 161-170 (1982)] durchgeführt, entsprechend
einer Modifikation von Gubler und Hoffman [Gubler und Hoffman,
Gene, 25: 263-269 (1983)]. Für die cDNA Synthese werden ungefähr
100-500 ng of Poly(A+) RNA aus immunpräzipitierten Polysomen
verwendet. Die reverse Transkription der mRNA in die entsprechende
cDNA erfolgt in einer Reaktionseinheit von 40 µl, die 50 mM Tris-HCl
pH 8,3, 100 mM NaCl, 10 mM $MgCl_2$, 10 mM DTT, 4 mM Natriumpyrophosphat, 1,25 mM der vier dNTP's, 1800 U/ml RNAsin (Promega Biotec),
100 µg/ml Oligo-$(dT)_{12-18}$ (Pharmacia/PL) und 3000 U/ml Reverse
Transkriptase (Molecular Genetics) enthält.

Das Reaktionsgemisch wird für einen Zeitraum von 25 Minuten bei
einer Temperatur von 43°C inkubiert. Anschliessend wird die Reaktion
durch Zugabe von 2 µl 0,5 M EDTA beendet. Das Reaktionsgemisch wird
mit einem Volumenteil Phenol:Chloroform extrahiert und die wässrige
Phase anschliessend mit einem halben Volumenteil Chloroform rückextrahiert. Auch die organische Phase wird erneut rückextrahiert und
zwar mit TE-Puffer.

Die wässrigen Phasen werden anschliessend vereinigt. Nach Zugabe von
einem Volumenteil 4 M Ammoniumacetat werden die Nucleinsäuren durch
Hinzufügen von zwei Volumenteilen Ethanol präzipitiert und anschliessend 20-30 Minuten auf Trockeneis kaltgestellt. Die Lösung
wird dann 5 Minuten auf Zimmertemperatur erwärmt und 15 Minuten in
einer Eppendorf-Zentrifuge ("Microfuge") bei einer Temperatur von
4°C zentrifugiert. Das so erhaltene Pellet wird in 25 µl TE-Puffer
aufgelöst. Der Vorgang der Nucleinsäure-Präzipitation und Wiedergewinnung wird, wie zuvor beschrieben, durch Zugabe von Ammoniumacetat
(25 µl; 4 M) und 100 µl Ethanol wiederholt. Das so erhaltene Pellet
wird dann mit 70 %igem Ethanol gewaschen, getrocknet und in 20 µl
Wasser gelöst.

Der Austausch des mRNA Stranges des mRNA:cDNA-Hybrids wird in einer
Reaktionseinheit von 50 µl durchgeführt, die 20 mM Tris-HCl pH 7,5,
5 mM $MgCl_2$, 10 mM Ammoniumsulfat, 100 mM KCl, 0,15 mM beta-NAD,
0,04 mM der vier dNTP's, 20 µl des Erststrang-Produkts, 10-20 µCie
$^{32}$P-dATP, 10 U/ml E.coli DNA Ligase (New England Biolabs), 230 U/ml
DNA Polymerase (Boehringer Mannheim) und 8,5 U/ml E.coli RNAse H
(Pharmacia/PL) enthält. Die Inkubationszeit beträgt 90 Minuten bei
einer Temperatur von 12° bis 14°C, sowie eine weitere Stunde bei
Raumtemperatur. Die doppelsträngige cDNA wird durch Phenol:Chloroform Extraktion gereinigt und durch Ethanol-Präzipitation wiedergewonnen, entsprechend der Beschreibung für das Erststrang-Produkt.
Das zuletzt erhaltene getrocknete Pellet wird in 20 µl Wasser
gelöst.

10 µl der Doppelstrang-cDNA werden in einem Gesamtvolumen von 20 µl,
enthaltend 100 mM Kaliumkakodylat pH 7,0, 1 mM $CoCl_2$, 0,2 mM DTT,
0,1 mM dCTP und 500 U/ml terminale Deoxynucleotid Transferase
(Pharmacia/PL) mit einem dCTP Anhang versehen. Die Inkubationszeit
beträgt 1 bis 2 Minuten bei einer Temperatur von 37°C. Anschliessend
werden 20 µl 4 mM EDTA hinzugefügt und das Enzym durch 10-minütige
Inkubation bei 65°C Hitze-inaktiviert.

PstI-verdaute und mit einem dG-Anhang versehene pBR322 (Bethesda
Research Labs, Inc.) wird in einem molaren Verhältnis von ca. 1:1 zu
der dC-haltigen, doppelsträngigen cDNA hinzugegeben (d.h. mit einem
etwa 5-10-fachen Ueberschuss im Hinblick auf das Molekulargewicht
des Vektors im Vergleich zu der angenommenen cDNA-Menge). Die
DNA-haltige Lösung (cDNA + Vektor) wird bis zu einer DNA-Gesamtkonzentration von 0,5-2,0 ng/µl (0,5 ng sind optimal) in Gegenwart von
10 mM Tris-HCl pH 7,5, 1 mM EDTA und 150 mM NaCl verdünnt. Diese Mischung wird zunächst 5 Minuten bei 65°C inkubiert, anschliessend erfolgt die eigentliche Renaturierungsreaktion ("annealing") bei einer Temperatur von 55-58°C über einen Zeitraum von 90 Minuten.

Der E.coli Stamm MM294 wird mit dem renaturierten cDNA:Vektor-Komplex transformiert unter Verwendung von 5 µl DNA pro 200 µl Aliquot an transformationskompetenten Zellen [Hanahan, J. Mol. Biol., 155: 557-580 (1983)]. Die Transformationsfrequenz der Kontrolle liegt bei $1-2 \times 10^8$ Transformanten pro µg kovalent geschlossener zirkulärer pBR322 DNA. Die transformierten Zellen werden auf LM-Platten (ohne Magnesium), die 17 µg/ml Tetrazyklin enthalten, ausplattiert (Hanahan, supra). Die so erhaltenen Kolonien werden auf Ampicillinempfindlichkeit hin untersucht. Die Tetrazyklin-resistenten und Ampillicin-sensitiven (ca. 50 %) Kolonien werden für die weitere Analyse herausgesucht.

### Hybrid-selektierte in vitro Translation von pGTR200

Plasmid DNA-Moleküle von 354 Ampillicin-sensitiven Transformanten werden unter Anwendung eines alkalischen Lyseverfahrens [Birnboim and Doly, Nucleic Acid Research, 7: 1513-1523 (1979)] gereinigt. Die gereinigten DNA-Moleküle werden anschliessend zur Bestimmung der Grösse der integrierten cDNA-Moleküle mit PstI verdaut. Von diesen enthielten nach der Durchführung einer Agarose-Gel-Elektrophorese insgesamt 134 sichtbare cDNA-Inserts. Inserts mit mehr als 800 Nucleotiden werden mit Hilfe der Southern blot Hybridisierung einer weitergehenden Analyse unterzogen [Southern, J. Mol. Biol., 98: 503-517 (1975)] unter Verwendung von $Y_a$ (pGTR261) und $Y_c$ (pGTR262) als Probemoleküle [Lai et al., J. Biol. Chem., 259: 5536-5542 (1984); Tu et al., J. Biol. Chem., 259: 9434-9439 (1984)].

12 Klone, die nicht mit diesen Probemolekülen hybridisieren, werden anschliessend mit Hilfe der Hybrid-selektierten in vitro Translation [Cleveland et al., Cell, 20: 95-105 (1980)] weiter charakterisiert. Einer dieser negativen Klone wird mit pGTR200 bezeichnet. Poly(A+)-RNA-Moleküle aus Rattenleber, die durch pGTR200-DNA, welche an aktivierter Aminophenylthioether Cellulose (APT-Papier) immobili-

siert vorliegt, selektioniert wurden, werden bei 75°C und 100°C
eluiert und anschliessend für die in vitro Translation im Kaninchen
Retikulozyten Lysat-System verwendet. Die in vitro Translationsprodukte werden mit Hilfe von Antisera gegen die Gesamt-Rattenleber-
GSTen immumpräzipitiert, gefolgt von einer Dodecylsulfat-Polyacrylamid Gelelektrophorese. Das immunpräzipitierte Produkt besitzt die
gleiche Mobilität wie $Y_b$. Durch pGTR200 wird keine andere Klasse von
GST-Untereinheiten selektiert. Früher durchgeführte Hybrid-selektierte in vitro Translationsexperimente mit $Y_a$ und $Y_c$ Klonen
erbrachten keine Produkte mit $Y_b$-Untereinheiten (Lai et al., supra;
Tu et al., supra).

### Nucleotid-Sequenz des pGTR200-DNA-Insert

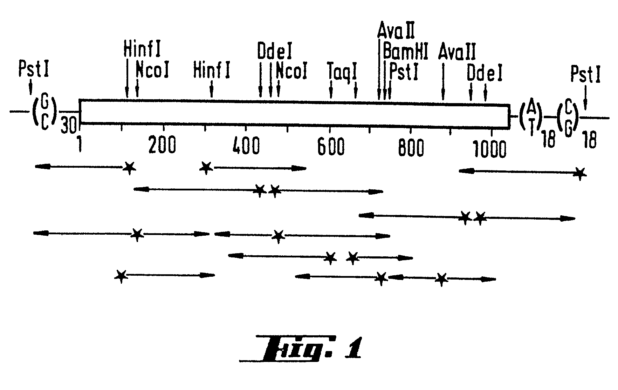

Die DNA Sequenz der cDNA in pGTR200 wird entsprechend der in
Abbildung 1 wiedergegebenen Strategie mit Hilfe des chemischen
Verfahrens nach Maxam und Gilbert [Maxam and Gilbert, Methods
Enzymol., 65: 499-560 (1980)] durchgeführt. Die DNA Fragmente, die
aufgrund der Spaltung mit verschiedenen Restriktionsenzymen erhalten
werden, werden am 3'-Ende markiert. Jede Bestimmung wurde mindestens
einmal wiederholt.

Die Nucleotidsequenz ist unten wiedergegeben. Für die 218 Reste des
offenen Leserahmens wurde der Einbuchstaben-Code für Aminosäuren
verwendet [Old and Primrose, Principles of Gene Manipulation,
(1985), Blackwell's Publications, London, S. 346]. Das Poly(A)-
Additionssignal, AATAAA ist unterstrichen.

```
        10        20        30        40        50        60
CTGAAGCCAAATTGAGAAGACCACAGCGCCAGAACCATGCCTATGATACTGGGATACTGG
                                        M  P  M  I  L  G  Y  W
        70        80        90       100       110       120
AACGTCCGCGGGCTGACACACCCGATCCGCCTGCTCCTGGAATACACAGACTCAAGCTAT
 N  V  R  G  L  T  H  P  I  R  L  L  L  E  Y  T  D  S  S  Y
       130       140       150       160       170       180
GAGGAGAAGAGATACGCCATGGGCGACGCTCCCGACTATGACAGAAGCCAGTGGCTGAAT
 E  E  K  R  Y  A  M  G  D  A  P  D  Y  D  R  S  Q  W  L  N
       190       200       210       220       230       240
GAGAAGTTCAAACTGGGCCTGGACTTCCCCAATCTGCCCTACTTAATTGATGGATCGCGC
 E  K  F  K  L  G  L  D  F  P  N  L  P  Y  L  I  D  G  S  R
```

```
          250       260       270       280       290       300
AAGATTACCCAGAGCAATGCCATAATGCGCTACCTTGCCCGCAAGCACCACCTGTGTGGA
 K   I   T   Q   S   N   A   I   M   R   Y   L   A   R   K   H   H   L   C   G

          310       320       330       340       350       360
GAGACAGAGGAGGAGCGGATTCGTGCAGACATTGTGGAGAACCAGGTCATGGACAACCGC
 E   T   E   E   E   R   I   R   A   D   I·  V   E   N   Q   V   M   D   N   R

          370       380       390       400       410       420
ATGCAGCTCATCATGCTTTGTTACAACCCCGACTTTGAGAAGCAGAAGCCAGAGTTCTTG
 M   Q   L   I   M   L   C   Y   N   P   D   F   E   K   Q   K   P   E   F   L

          430       440       450       460       470       480
AAGACCATCCCTGAGAAGATGAAGCTCTACTCTGAGTTCCTGGGCAAGCGACCATGGTTT
 K   T   I   P   E   K   M   K   L   Y   S   E   F   L   G   K   R   P   W   F

          490       500       510       520       530       540
GCAGGGGACAAGGTCACCTATGTGGATTTCCTTGCTTATGACATTCTTGACCAGTACCAC
 A   G   D   K   V   T   Y   V   D   F   L   A   Y   D   I   L   D   Q   Y   H

          550       560       570       580       590       600
ATTTTTGAGCCCAAGTGCCTGGACGCCTTCCCAAACCTGAAGGACTTCCTGGCCCGCTTC
 I   F   E   P   K   C   L   D   A   F   P   N   L   K   D   F   L   A   R   F

          610       620       630       640       650       660
GAGGGCCTGAAGAAGATCTCTGCCTACATGAAGAGCAGCCGCTACCTCTCAACACCTATA
 E   G   L   K   K   I   S   A   Y   M   K   S   S   R   Y   L   S   T   P   I

          670       680       690       700       710       720
TTTTCGAAGTTGGCCCAATGGAGTAACAAGTAGGCCCTTGCTACACTGGCACTCACAGAG
 F   S   K   L   A   Q   W   S   N   K   *

          730       740       750       760       770       780
AGGACCTGTCCACATTGGATCCTGCAGGCACCCTGGCCTTCTGCACTGTGGTTCTCTCTC

          790       800       810       820       830       840
CTTCCTGCTCCCTTCTCCAGCTTTGTCAGCCCCATCTCCTCAACCTCACCCCAGTCATGC

          850       860       870       880       890       900
CCACATAGTCTTCATTCTCCCCACTTTCTTTCATAGTGGTCCCCTTCTTTATTGACACCT

          910       920       930       940       950       960
TAACACAACCTCACAGTCCTTTTCTGTGATTTGAGGTCTGCCCTGAACTCAGTCTCCCTA

          970       980       990      1000      1010      1020
GACTTACCCCAAATGTAACACTGTCTCAGTGCCAGCCTGTTCCTGGTGGGGGAGCTGCCC

         1030      1040      1050      1060      1070
CAGGCCTGTCTCATCTTTAATAAAGCCTGAAACACAAAAAAAAAAAAAAAAAAA
```

<u>Beispiel IB: Isolierung des partiellen GST-cDNA-Klons Y$_b$187</u>

Mit Hilfe der im Prinzip gleichen Methode erhält man den cDNA-Klon Y$_b$187. Bei Y$_b$187 handelt es sich um einen partiellen cDNA-Klon, dem am 5'-Ende der codierenden Sequenz 96 Nucleotide fehlen. Die

Nucleotid- und die entsprechende Aminosäure-Sequenz sowie die Sequenz für die 96 fehlenden Nucleotide und für $Y_b 187$ sind im folgenden wiedergegeben:

```
                1 0                           30
     ATG CCT ATG ACA CTG GGT TAC TGG GAC ATC CGT GGG CTG GCT CAC
     Met Pro Met Thr Leu Gly Tyr Trp Asp Ile Arg Gly Leu Ala His

         50                           7 0                    90
     GCC ATT CGC CTG TTC CTG GAG TAT ACA GAC ACA AGC TAT GAG GAC
     Ala Ile Arg Leu Phe Leu Glu Tyr Thr Asp Thr Ser Tyr Glu Asp

                        1 10                       13 0
     AAG AAG TAC AGC ATG GGG GAT GCT CCC GAC TAT GAC AGA AGC CAG
     Lys Lys Tyr Ser Met Gly Asp Ala Pro Asp Tyr Asp Arg Ser Gln

                   150                        1 70
     TGG CTG AGT GAG AAG TTC AAA CTG GGC CTG GAC TTC CCC AAT CTG
     Trp Leu Ser Glu Lys Phe Lys Leu Gly Leu Asp Phe Pro Asn Leu

              19 0                      210
     CCC TAC TTA ATT GAT GGG TCA CAC AAG ATC ACC CAG AGC AAT GCC
     Pro Tyr Leu Ile Asp Gly Ser His Lys Ile Thr Gln Ser Asn Ala

       2 30                      25 0                     270
     ATC CTG CGC TAC CTT GGC CGG AAG CAC AAC CTT TGT GGG GAG ACA
     Ile Leu Arg Tyr Leu Gly Arg Lys His Asn Leu Cys Gly Glu Thr

                        2 90                       31 0
     GAG GAG GAG AGG ATT CGT GTG GAC GTT TTG GAG AAC CAG GCT ATG
     Glu Glu Glu Arg Ile Arg Val Asp Val Leu Glu Asn Gln Ala Met

                   330                          3 50
     GAC ACC CGC CTA CAG TTG GCC ATG GTC TGC TAC AGC CCT GAC TTT
     Asp Thr Arg Leu Gln Leu Ala Met Val Cys Tyr Ser Pro Asp Phe

              37 0                        390
     GAG AGA AAG AAG CCA GAG TAC TTA GAG GGT CTC CCT GAG AAG ATG
     Glu Arg Lys Lys Pro Glu Tyr Leu Glu Gly Leu Pro Glu Lys Met

       4 10                       43 0                      450
     AAG CTT TAC TCC GAA TTC CTG GGC AAG CAG CCA TGG TTT GCA GGG
     Lys Leu Tyr Ser Glu Phe Leu Gly Lys Gln Pro Trp Phe Ala Gly

                        4 70                       49 0
     AAC AAG ATT ACG TAT GTG GAT TTT CTT GTT TAC GAT GTC CTT GAT
     Asn Lys Ile Thr Tyr Val Asp Phe Leu Val Tyr Asp Val Leu Asp

                   510                        5 30
     CAA CAC CGT ATA TTT GAA CCC AAG TGC CTG GAC GCC TTC CCA AAC
     Gln His Arg Ile Phe Glu Pro Lys Cys Leu Asp Ala Phe Pro Asn

              55 0                        570
     CTG AAG GAC TTC GTG GCT CGG TTT GAG GGC CTG AAG AAG ATA TCT
     Leu Lys Asp Phe Val Ala Arg Phe Glu Gly Leu Lys Lys Ile Ser

       5 90                         61 0                    630
     GAC TAC ATG AAG AGC GGC CGC TTC CTC TCC AAG CCA ATC TTT GCA
     Asp Tyr Met Lys Ser Gly Arg Phe Leu Ser Lys Pro Ile Phe Ala
```

0256223

```
                          6 50
AAG ATG GCC TTT TGG AAC CCA AAG TAG
Lys Met Ala Phe Trp Asn Pro Lys End
```

Beispiel IC: Der vollständige, Y_b 187 entsprechende Klon

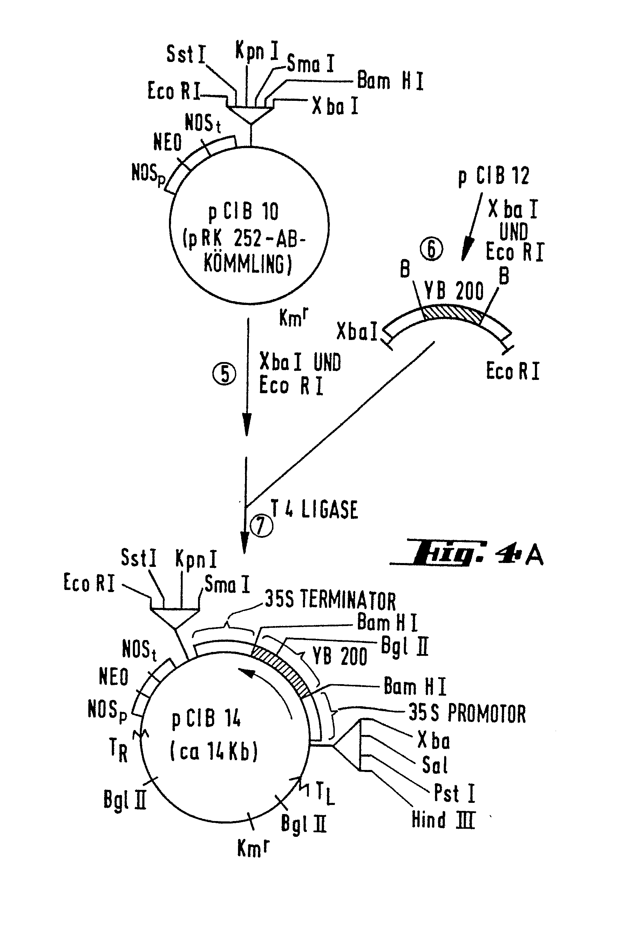

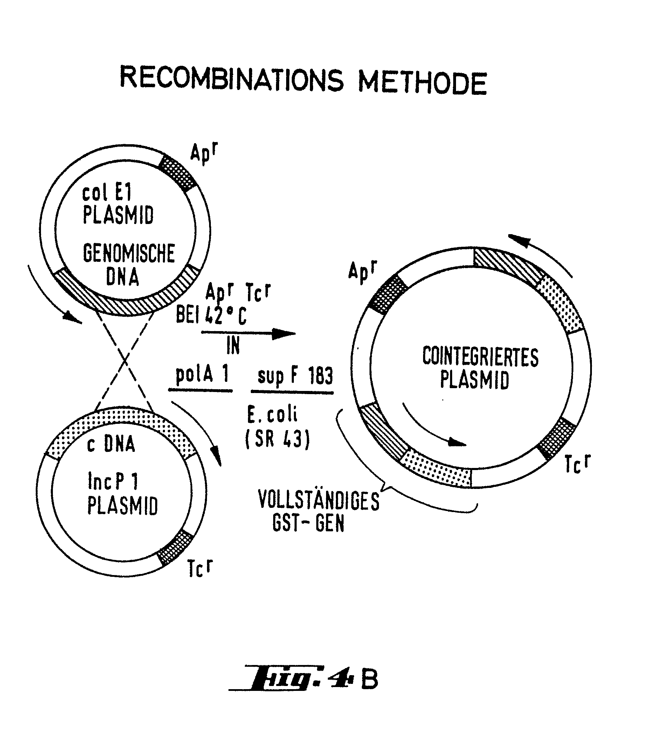

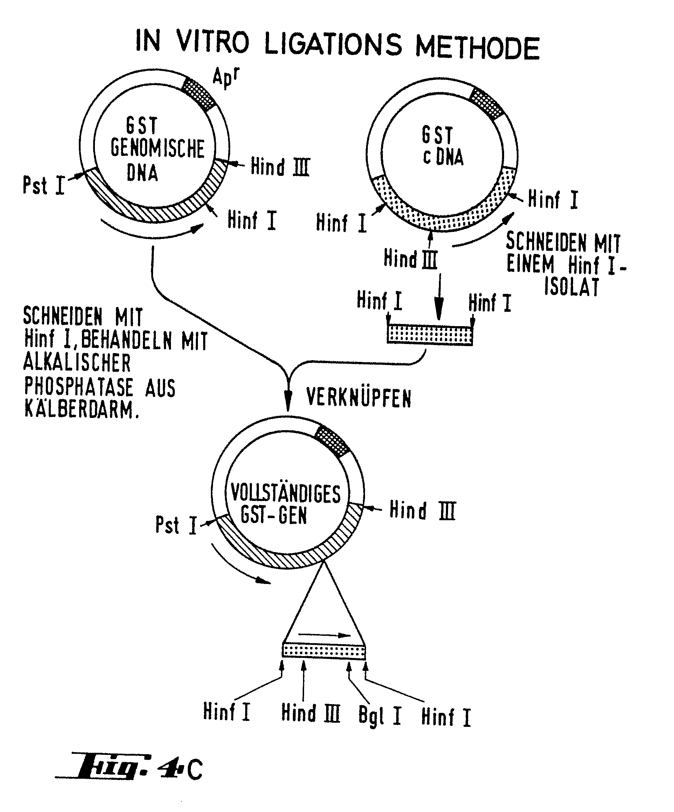

Die fehlenden Nucleotide werden entweder in vivo durch Rekombination oder in vitro durch Verknüpfung der entsprechenden Restriktionsfragmente zu Y_b 187 hinzugefügt. Diese Verfahren sind in den Abbildungen 4B und 4C dargestellt.

Eine Rekombination kann durch die Einführung eines genomischen DNA Klons, als Bestandteil des Plasmids colEl (z.B. pUC8 oder pBR322) und eines cDNA Klons, der sich auf dem Plasmid inc Pl (z.B. pRK290) befindet, in den E.coli Stamm SR43 (ATCC Hinterlegungsnummer 67217, hinterlegt am 22. September 1986) durchgeführt werden. Der Stamm SR43 trägt die Mutationen polAl supF183 [Tacon, W. & Sherratt, D., Mol. Gen. Genet., 147: 331-335 (1976)], so dass die Replikation des colEl-Plasmids bei einer restriktiven Temperatur (42°C) gehemmt ist.

Eine Ueberführung der SR43-Bakterien, welche die beiden genannten Plasmide enthalten, von einer permissiven Temperatur von 28°C auf 42°C unter gleichzeitiger Beibehaltung der Selektionierbarkeit auf Antibiotikaresistenz, die auf dem Plasmid-colEl (z.B. Ap-Resistenz von pBR322) liegt, gestattet die Selektion von Bakterien, die eine kointegrierte Form der zwei Plasmide enthalten.

Solche kointegrierten Plasmide kommen durch Rekombination homologer DNA Regionen zustande.

Die Isolierung kointegrierter Plasmide ermöglicht die Klonierung der Gesamt cDNA Sequenz sowie der stromaufwärts gelegenen genomischen DNA in Form eines einzigen PstI oder BamHI Fragments.

Restriktion der stromaufwärts gelegenen DNA mit Bal31, gefolgt von der Addition eines BamHI-Linkers, gestattet die Isolierung von Klonen, die die gesamte kodierende Region (bestätigt durch DNA-Sequenz-Analyse) enthalten.

Die gesamte kodierende Region wird in Form eines BamHI-Fragments in pCIB710 kloniert, entsprechend der Beschreibung in Beispiel IA, und für die Uebertragung auf die Pflanzenzelle verwendet.

Dasselbe Fragment wird für die Expression in E.coli in pDR540 kloniert, wie in Beispiel IV, unten, beschrieben.

Eine andere Möglichkeit für die Konstruktion eines vollständigen Klones, der $Y_b187$ entspricht, besteht in der Verknüpfung geeigneter Restriktionsfragmente der cDNA und der genomischen Klone (Abbildung I).

Das Plasmid pUC19 enthält das 5'-Ende des genomischen Klons, eingespleisst als ein PstI-HindIII-Fragment, welches annäherungsweise die ersten 400 Basen der kodierenden Sequenz enthält.

Das 3'-Ende der kodierenden Sequenz der cDNA kann als ein 630 Basen umfassendes HinfI-Fragment von dem partiellen cDNA-Klon in BR325 isoliert werden. Das an seiner einzigen HinfI-Stelle geschnittene pUC Plasmid und das HinfI Fragment, welches das 3'-Ende des Gens trägt, werden miteinander verknüpft, wobei die gesamte kodierende Sequenz des GST-Gens wiederhergestellt wird. Das komplette Gen wird als ein BglI-PstI-Fragment isoliert.

Eine Isolierung der gesamten kodierenden Region, ohne die stromaufwärts gelegenen DNA Sequenzen, lässt sich durch Herausschneiden der stromaufwärts gelegenen DNA mit Hilfe von Bal31 gefolgt von einer BamHI Linker Addition erreichen. Dieses BamHI-Fragment wird dann in pCIB710 bzw. in pDR540 eingespleisst, entsprechend den oben gemachten Angaben.

**Beispiel ID:** <u>Hybride Klone</u>

Hybride Klone, die sich aus kodierenden Sequenzen für verschiedene Gene herleiten, werden im Prinzip in der gleichen Art und Weise konstruiert, wie dies zuvor für die Kombination des partiellen cDNA Klons $Y_b 187$ und der fehlenden Nucleotide beschrieben wurde (Beispiel IC), indem heterologe Gene als Ausgangsmaterial für die zwei DNA-Fragmente verwendet werden.

**Beispiel IE:** <u>Isolierung des GST-cDNA Klons $GlY_b$</u>

Mit Hilfe des Phagen-Expressionsvektors λ gt11 [Young, R.A. and Davis, R.W., <u>Proc. Natl. Acad. Sci. USA</u>, <u>80</u>: 1194 (1983)] wird eine cDNA-Genbibliothek hergestellt, ausgehend von einer poly(A) RNA, die zuvor aus Rattengehirn isoliert wurde gemäss den oben beschriebenen Verfahren (siehe Beispiel IA).

Die cDNA wird anschliessend mit Hilfe eines Antikörper-Screenings unter Verwendung von Antikörpern, die gegen GST aus dem Rattengehirn gerichtet sind, isoliert.

Die Isolierung der RNA sowie die Herstellung und Isolierung der zugehörigen cDNA erfolgt nach bereits bekannten Verfahren, wie sie z.B. bei Young und Davis beschrieben sind [Young, R.A. and Davis, R.W., <u>Science</u>, <u>222</u>: 778-782 (1983)].

Die Nucleotidsequenz und die zugehörige Aminosäuresequenz des resultierenden cDNA Klons $GlY_b$ sind im folgenden wiedergegeben:

```
              10                          30
  A GAC CCC AGC ACC ATG CCC ATG ACA CTG GGT TAC TGG GAC ATC
                  Met Pro Met Thr Leu Gly Tyr Trp Asp Ile

      5 0                          70
  CGT GGG CTA GCG CAT GCC ATC CGC CTG CTC CTG GAA TAC ACA GAC
  Arg Gly Leu Ala His Ala Ile Arg Leu Leu Leu Glu Tyr Thr Asp

  90                      11 0                      130
  TCG AGC TAT GAG GAG AAG AGA TAC ACC ATG GGA GAC GCT CCC GAC
  Ser Ser Tyr Glu Glu Lys Arg Tyr Thr Met Gly Asp Ala Pro Asp

              1 50                         17 0
  TTT GAC AGA AGC CAG TGG CTG AAT GAG AAG TTC AAA CTG GGC CTG
  Phe Asp Arg Ser Gln Trp Leu Asn Glu Lys Phe Lys Leu Gly Leu
```

```
                  190                                  2 10
GAC TTC CCC AAT CTG CCC TAC TTA ATT GAT GGA TCA CAC AAG ATC
Asp Phe Pro Asn Leu Pro Tyr Leu Ile Asp Gly Ser His Lys Ile

          23 0                              250                  2
ACC CAG AGC AAT GCC ATC CTG CGC TAT CTT GGC CGC AAG CAC AAC
Thr Gln Ser Asn Ala Ile Leu Arg Tyr Leu Gly Arg Lys His Asn

70                              29 0                        310
CTG TGT GGG GAG ACA GAA GAG GAG AGG ATT CGT GTG GAC ATT CTG
Leu Cys Gly Glu Thr Glu Glu Glu Arg Ile Arg Val Asp Ile Leu

              3 30                              35 0
GAG AAT CAG CTC ATG GAC AAC CGC ATG GTG CTG GCG AGA CTT TGC
Glu Asn Gln Leu Met Asp Asn Arg Met Val Leu Ala Arg Leu Cys

          370                              3 90
TAT AAC CCT GAC TTT GAG AAG CTG AAG CCA GGG TAC CTG GAG CAA
Tyr Asn Pro Asp Phe Glu Lys Leu Lys Pro Gly Tyr Leu Glu Gln

      41 0                              430                      4
CTG CCT GGA ATG ATG CGG CTT TAC TCC GAG TTC CTG GGC AAG CGG
Leu Pro Gly Met Met Arg Leu Tyr Ser Glu Phe Leu Gly Lys Arg

50                              47 0                        490
CCA TGG TTT GCA GGG GAC AAG ATC ACC TTT GTG GAT TTC ATT GCT
Pro Trp Phe Ala Gly Asp Lys Ile Thr Phe Val Asp Phe Ile Ala

                  5 10                          53 0
TAC GAT GTT CTT GAG AGG AAC CAA GTG TTT GAG GCC ACG TGC CTG
Tyr Asp Val Leu Glu Arg Asn Gln Val Phe Glu Ala Thr Cys Leu

              550                              5 70
GAC GCG TTC CCA AAC CTG AAG GAT TTC ATA GCG CGC TTT GAG GGC
Asp Ala Phe Pro Asn Leu Lys Asp Phe Ile Ala Arg Phe Glu Gly

      59 0                              610                      6
CTG AAG AAG ATC TCC GAC TAC ATG AAG TCC AGC CGC TTC CTC CCA
Leu Lys Lys Ile Ser Asp Tyr Met Lys Ser Ser Arg Phe Leu Pro

30                              65 0                        670
AGA CCT CTG TTC ACA AAG ATG GCT ATT TGG GGC AGC AAG TAG GAC
Arg Pro Leu Phe Thr Lys Met Ala Ile Trp Gly Ser Lys End Asp

                  6 90                          71 0
CCT GAC AGG TGG GCT TTA GGA GAA AGA TAC CAA ATC TCC TGG GTT
Pro Asp Arg Trp Ala Leu Gly Glu Arg Tyr Gln Ile Ser Trp Val

              730                              7 50
TGC CAA GAG CCC TAA GGA GCG GGC AGG ATT CCT GAG CCC CAG AGC
Cys Gln Glu Pro End Gly Ala Gly Arg Ile Pro Glu Pro Gln Ser

          77 0                              790                  8
CAT GTT TTC TTC CTT CCT TCC ATT CCA GTC CCC AAG CCT TAC CAG
His Val Phe Phe Leu Pro Ser Ile Pro Val Pro Lys Pro Tyr Gln

10                              83 0                        850
CTC TCA TTT TTT GGT CAT CAA ATT CCT GCC AAA CAC AGG CTC TTA
Leu Ser Phe Phe Gly His Gln Ile Pro Ala Lys His Arg Leu Leu
```

```
                 8 70                          89 0
AAA GCC CTA GCA ACT CCT TTC CAT TAG CAA AAT AGC CTT CTA AAG
Lys Ala Leu Ala Thr Pro Phe His End Gln Asn Ser Leu Leu Lys

                 910                          9 30
TTA AAG TGC CCC GCC CCC ACC CCT CGA GCT CAT GTG ATT GGA TAG
Leu Lys Cys Pro Ala Pro Thr Pro Arg Ala His Val Ile Gly End

        95 0                          970                      9
TTG GCT CCC AAC ATG TGA TTA TTT TGG GCA GGT CAG GCT CCC CGG
Leu Ala Pro Asn Met End Leu Phe Trp Ala Gly Gln Ala Pro Arg

90                            101 0                  1 030
CAG ATG GGG TCT ATC TGG AGA CAG TAG ATT GCT AGC AGC TTT GAC
Gln Met Gly Ser Ile Trp Arg Gln End Ile Ala Ser Ser Phe Asp

                      10 50                    107 0
CAC CGT AGC CAA GCC CCT CTT CTT GCT GTT TCC CGA GAC TAG CTA
His Arg Ser Gln Ala Pro Leu Leu Ala Val Ser Arg Asp End Leu

            1 090                      11 10
TGA GCA AGG TGT GCT GTG TCC CCA GCA CTT GTC ACT GCC TCT GTA
End Ala Arg Cys Ala Val Ser Pro Ala Leu Val Thr Ala Ser Val

        113 0                  1 150                      11
ACC CGC TCC TAC CGC TCT TTC TTC CTG CTG CTG TGA GCT GTA CCT
Thr Arg Ser Tyr Arg Ser Phe Phe Leu Leu Leu End Ala Val Pro

70                      119 0                  1 210
CCT GAC CAC AAA CCA GAA TAA ATC ATT CTC CCC TTA AAA AAA AAA
Pro Asp His Lys Pro Glu End Ile Ile Leu Pro Leu Lys Lys Lys

AAA AAA AAA A
Lys Lys Lys
```

## Beispiel II: Konstruktion des Plasmids pCIB710

Das Plasmid pLW111, ATCC Nr. 40235, besteht aus den drei kleineren
EcoRI-Fragmenten des BJI-Stammes von Cauliflower Mosaik Virus (CaMV)
[Franck et al., Cell, 21: 285-294 (1980)], die in pMB9 kloniert
werden. Das Plasmid pLW111 wird mit BglII verdaut und ein 1149 Bp
umfassendes Fragment (Basenpaare #6494-7643) isoliert. Dieses
Fragment wird in die BamHI-Stelle von pUC19 eingespleisst. Dieses
Restriktionsfragment kodiert sowohl für den Promotor der CaMV 35S
RNA als auch für das PolyA Additionssignal (d.h. den Terminator) für
dieses Transkript.

## In Vitro Mutagenese

Zwischen diesem Promotor und Terminator wird via in vitro Mutagenese eine einzelne BamHI-Restriktionsschnittstelle eingefügt. Es wird ein 36-Basen umfassendes Oligonucleotid synthetisiert, welches, abgesehen von einer BamHI-Restriktionsstelle, die bei dem Basenpaar #7464 in die Sequenz eingebaut ist, mit der entsprechenden Sequenz der CaMV-Region identisch ist.

Das oben erwähnte 1149 Bp umfassende BglII Fragment wird zunächst in M13mp19 kloniert; anschliessend wird eine einzelsträngige Phagen-DNA isoliert. Diese Einzelstrang-DNA wird dann mit dem synthetischen Oligonucleotid gepaart ("annealed").

Unter Verwendung dieses Oligonucleotids als Primer wird dann zunächst ein neuer DNA-Strang synthetisiert und die DNA anschliessend in den E.coli Stamm JM101 |Zoller & Smith, DNA 3: 479-488 (1980)| mittels Transfektion eingeschleust.
Die Isolierung des M13-Phagen mit der eingebauten BamHI-Stelle wird entsprechend der Beschreibung bei Zoller & Smith, supra, durchgeführt.

## Selektion des gewünschten Mutanten-Phagen

Das 36-Basen-Oligonucleotid wird in einer Kinase-Reaktion mit $^{32}$P-ATP markiert. Eine Auswahl von durch Transfektion erhaltener M13 Phagen-Plaques wird auf einen Nitrocellulose-Filter übertragen. Dieser Filter wird mit dem markierten 36 Basen umfassenden Oligonucleotid hybridisiert, und anschliessend bei steigenden Temperaturen gewaschen. Das markierte Oligonucleotid, gebunden an den Mutanten-Phagen, ist noch bei höheren Waschtemperaturen stabil. Einer dieser bei erhöhter Temperatur stabilen Phagen wird isoliert und zur Bestätigung der Anwesenheit der BamHI-Stelle sequenziert.

## Konstruktion von pCIB710

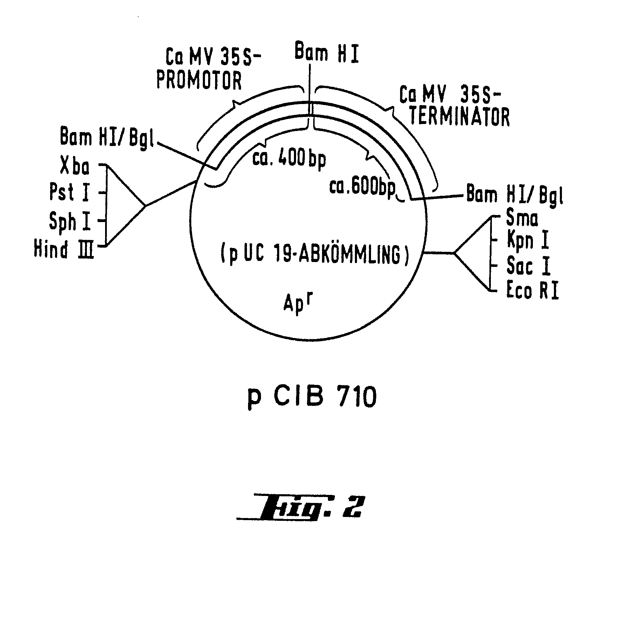

Von diesem Phagen isolierte doppelsträngige DNA wird mit HindIII und EcoRI verdaut. pUC19 wird ebenfalls mit HindIII und EcoRI gespalten. Diese beiden verdauten DNA-Moleküle werden anschliessend miteinander verknüpft. Transformanten werden aufgrund ihrer Ampicillin-Resistenz
selektiert. Einer der Transformanten, die aus dieser Verknüpfungsreaktion hervorgehen, ist pCIB710, ein Plasmid, das in Abbildung 2
dargestellt ist.

Beispiel III: Konstruktion der Plasmide pCIB11, pCIB12, pCIB13
             und pCIB14

1. Das Plasmid pGTR200 wird mit HaeII, PstI und PvuII verdaut und
das 678 Bp umfassende HaeII/PstI-Fragment, das die GST kodierende
Sequenz enthält, aus einem Agarose-Gel isoliert.

2. Dieses HaeII/PstI Fragment erhält durch Behandlung mit T4 DNA-
Polymerase glatte Enden, an die mit Hilfe der T4 DNA-Ligase BamHI-
Linker (dCGGATCCG-New England Biolabs) angeknüpft werden.

3. Das Plasmid pCIB710 wird mit BamHI geschnitten und anschliessend
mit alkalischer Phosphotase aus Kälberdarm (Boehringer Mannheim)
behandelt.

4. Das oben beschriebene, mit einem BamHI-Linker versehene GST-
Fragment wird in das mit BamHI verdaute Plasmid pCIB710 eingespleisst, die Ligationsmischung in den E.coli Stamm HB101 transformiert und die gewünschten Transformanten anhand ihrer Ampillicin-
Resistenz selektiert. Es lassen sich sowohl Transformanten charakterisieren, welche die GST codierende Sequenz im Hinblick auf die
Transkription, ausgehend vom CaMV-Promotor, in der richtigen
Orientierung (pCIB12) enthalten, als auch solche mit einer entgegengesetzten Orientierung (pCIB11).

5. Das Plasmid pCIB10 (siehe Beispiel IV) wird mit Hilfe von XbaI
und EcoRI verdaut.

6. Das Plasmid pCIB12 wird ebenfalls mit Hilfe von XbaI und EcoRI
verdaut und das kleinere Fragment aus einem Agarose-Gel isoliert.

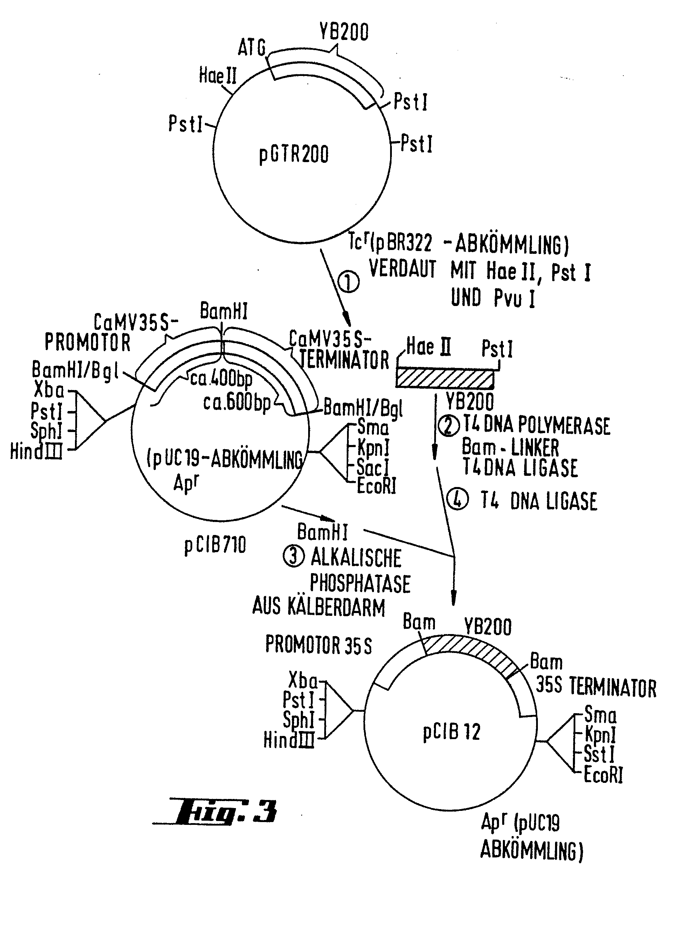

7. Das isolierte XbaI/EcoRI Fragment, welches das chimäre Gen trägt, wird in das zuvor verdaute Plasmid pCIB10 eingespleisst; das gesamte Ligationsgemisch wird anschliessend in E.coli HB101 transformiert. Die Transformanten werden aufgrund ihrer Kanamycin-Resistenz selektiert. Diese Transformanten, die die GST kodierende Sequenz in der passenden Orientierung im Hinblick auf die Transkription, ausgehend vom CaMV-Promotor, enthalten, werden mit pCIB14 (siehe Abbildung 3) bezeichnet.

8. Das Plasmid pCIB11 wird zur Konstruktion von pCIB13 in ähnlicher Weise manipuliert. Es handelt sich bei pCIB13 um ein Plasmid, welches die GST kodierende Region in einer Orientierung enthält, die einer geregelten Transkription, ausgehend vom CaMV-Promotor, entgegensteht.

Einführung von pCIB13 und pCIB14 in Agrobacterium
Gereinigte Plasmid-DNA vom Typ pCIB13 oder pCIB14 wird mit Hilfe der Transformation in Agrobacterium tumefaciens A136 eingeschleust |Watson et al., J. Bacteriol., 123: 255-264 (1975)|, welches das Plasmid pCIB542 (siehe ebenso Beispiel VII, unten) enthält. Die Transformanten werden auf Kanamycin (50 µg/ml) und auf Spectinomycin (25 µg/ml) selektiert.

Beispiel IV: Einbau des $Y_b$ 200 GST in den trp-lac-Expressions-Vektor
Das Plasmid pCIB12 wird mit BamHI verdaut und ein 708 Bp umfassendes, mit einem Bam-Linker versehenes GST-Genfragment isoliert. Das Plasmid pDR540 (Pharmacia P-L Biochemicals), ein trp-lac-Expressions-Vektor |Russel, D.R. and Bennett, G.N., Gene, 20: 231-243 (1982)| wird ebenfalls mit BamHI verdaut und das GST-Fragment in die entsprechende Restriktionsschnittstelle eingespleisst. Das resultierende rekombinante Plasmid wird in den E.coli Stamm JM103 transformiert. Kulturen des resultierenden Stammes sind zu jedem beliebigen Zeitpunkt durch Zugabe von 1,0 mM Isopropyl-beta-D-thiogalactosid (IPTG) induzierbar.

Nach Induktion durch IPTG und Expression des $Y_b200$ GST-Gens wird der bakterielle Wirt pelletiert. Das gebildete Pellet wird anschliessend in 59 ml 0,2 M Tris-HCl, pH 8,0, und 1 mM EDTA resuspendiert. Zu dieser Suspension werden 0,5 ml 100 mM Phenylmethylsulfonylfluorid (PMSF) in Ethanol und 5 ml Lysozym (10 mg/ml) in Pufferlösung hinzugefügt.

Die Suspension wird für ca. 15 Minuten bei einer Temperatur von 37°C inkubiert, bis die bakteriellen Zellen lysiert sind. Die in der Suspension vorliegenden lysierten Bakterienzellen werden erneut pelletiert. Der Ueberstand wird gesammelt und anschliessend gegen 25 mM Tris-HCl, pH 8,0, mit 1/100 Volumen PMSF dialysiert.

Der dialysierte Ueberstand wird dann auf GST-Aktivität hin untersucht. Anschliessend wird der dialysierte Ueberstand auf eine S-Hexylglutathion-agarose Affinitätssäule gepackt, bei einer Durchflussrate von 0,5 ml/Minute. Die Säule wird mit 25 mM Tris-HCl und 0,2 M KCl gewaschen bis das Absorptionsvermögen bei 280 nm unter einen Wert von 0,005 absinkt. Nachdem alles nicht gebundene Material von der Säule heruntergewaschen ist, wird das gebundene Material mit 25 mM Tris-HCl, 0,2 M KCl (pH 8,0) enthaltend 5 mM S-Hexylglutathion und 2,5 mM Glutathion, eluiert. Die eluierten Fraktionen werden anhand ihres Absorptionsvermögens bei 280 nm kontrolliert.

Die Fraktionen, von denen man annimmt, dass sie das gereinigte Enzym enthalten, werden einzeln gegen 0,1 M $NaPO_4$-Puffer (pH 6,5), der PEG zur Konzentrierung der Fraktionen enthält, und anschliessend gegen den gleichen Puffer ohne PEG, dialysiert.

Die Volumina in jeder der dialysierten Fraktionen werden registriert. Für jede Fraktion wird die Konzentration und die Menge der Probe kalkuliert und anschliessend in der Weise verdünnt, dass am Ende in jeder Fraktion der gleiche Anteil im Verhältnis zu der Ausgangsfraktion vorhanden ist. Jede Fraktion wird auf ihre Enzymaktivität hin untersucht.

Die Bestimmung der Enzymaktivität wird unter Verwendung von 1-Chlor-
2,4-dinitrobenzol (ClDNB) als Substrat durchgeführt. Für jede
Reaktion wird zu 5 mM reduziertem Glutathion (30,8 mg/ml in Puffer,
0,1 M Natriumphosphat, pH 6,5) und GST-Enzym 1 mM ClDNB (20,2 mg/ml
in Ethanol) zugegeben.

ClDNB und reduziertes Glutathion werden täglich frisch hergestellt.
Die Reaktionseinheit hat ein Endvolumen von 1 ml. Die Reaktion wird
bei Zimmertemperatur durchgeführt und durch Zugabe von ClDNB
gestartet. Der Reaktionsverlauf wird mit Hilfe eines Gilford
Spektrophotometers bei einer Wellenlänge von 340 nm überwacht. Ein
typischer Reaktionsablauf beinhaltet 10 bis 50 Einheiten GST, wobei
1 Einheit dem Umsatz von 1 mmol Substrat pro Minute und ml entspricht.

Die Proteinkonzentration der enzymatisch aktiven Fraktionen im
Ausgangsmaterial wird bestimmt (Biorad, BSA Standard). Jede der
enzymatisch aktiven Fraktionen wird auf einem 15 %igen Lämmli Gel
analysiert unter Verwendung von Verdünnungsreihen von Standard-
Enzymen (1,0 µg, 0,3 µg und 0,1 µg) als quantitative Standards. Der
Nachweis des gereinigten Enzyms erfolgt mit Hilfe des "Immunblotting" unter Verwendung spezifischer Antikörper.

Beispiel V:
Konstruktion von pCIB10 und pCIB10a
Die Konstruktion der Plasmide pCIB10 und pCIB10a ist in der hängigen
US-Patentanmeldung mit der Serial-Nummer 757.098, die am 19. Juli 1985
unter dem Titel "Transformation of Plastids and Mitochondria"
eingereicht wurde und die per Referenz hierin inkorporiert ist,
beschrieben.

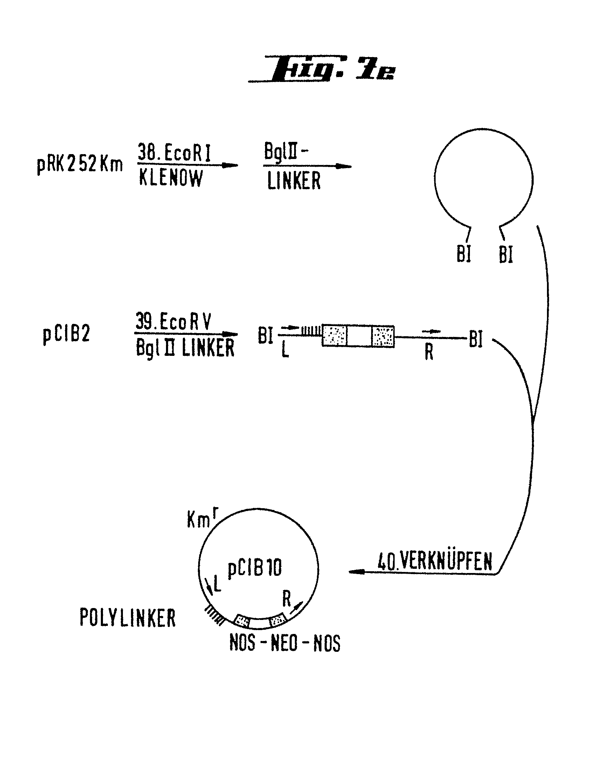

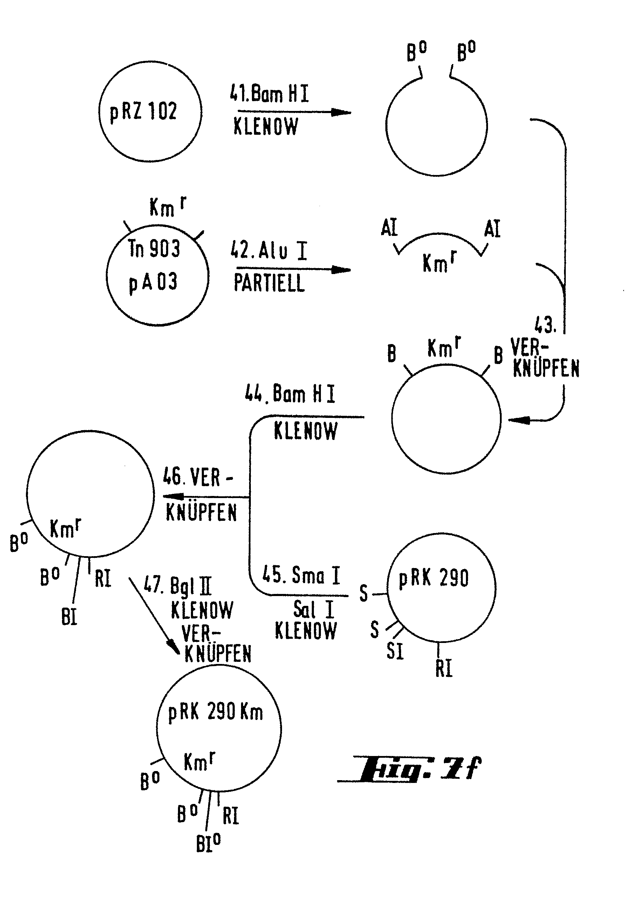

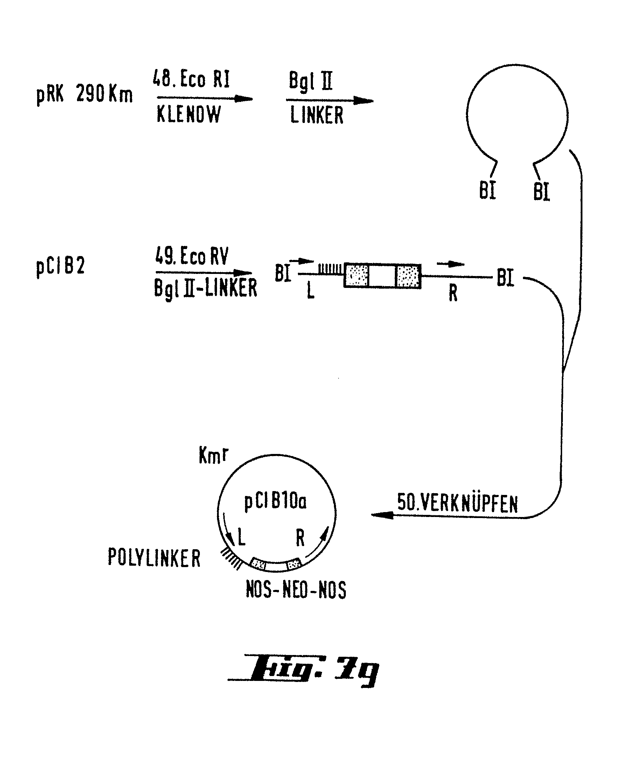

Die folgenden Einzelschritte, die in der oben genannten Referenz-
Patentanmeldung wie folgt beschrieben und numeriert wurden, werden
für die Konstruktion der Plasmide durchgeführt (siehe Abbildung 7a-g):

Konstruktion von pCIB10

15. Eine T-DNA Fragment, das die linke Grenzsequenz des Plasmids pTiT37 trägt, wird von pBR325 (EcoRI29) |Yadav et al., Proc. Natl. Acad. Sci. USA, 79: 6322 (1982)| isoliert. pBR325 (EcoRI29) wird mit EcoRI geschnitten und das 1,1 Kb umfassende Fragment mit HindIII-Linkern (New England Biolabs) verknüpft.

16. Das Plasmid pBR322 (Bolivar et al., Gene, 2: 75) wird mit HindIII geschnitten.

17. Das in Schritt 15 genannte, die linke Grenzsequenz enthaltende Fragment, wird in das HindIII verdaute Plasmid pBR322 eingespleisst.

18. Das Plasmid pBR322 mit der linken Grenzsequenz aus Schritt 17 wird mit ClaI und HindIII verdaut; das 1,1 Kb umfassende HindIII/ClaI Fragment wird isoliert |Hepburn et al., J. Mol. Appl. Genet., 2: 211 (1983)|.

19. Das Plasmid pUC18 |Norrander et al., Gene, 26: 101 (1983)| wird mit HindIII und EcoRI geschnitten; ein 60 Bb umfassender Polylinker wird mit Hilfe der T4 Polynucleotidkinase und Gamma $^{32}$P-dATP endmarkiert und aus einem Acrylamid-Gel isoliert.

20. Das Plasmid pBR322 wird mit EcoRI und ClaI geschnitten und das grosse Fragment isoliert.

21. Der 60 Bp umfassende HindIII/EcoRI Polylinker und das 1,1 Kb umfassende HindIII/ClaI Fragment von EcoRI29 werden in das Plasmid pBR322, das zuvor mit ClaI und EcoRI geschnitten wurde, eingespleisst, unter Bildung von PCIB5 (Schritt 15-21, siehe Abbildung 7a).

22. Aus Bin6 [Bevan, Nucleic Acids Res., 12: 8711 (1984)] wird ein chimäres Gen, das für Kanamycinresistenz kodiert (nos-neo-nos), in Form eines SalI/EcoRI Fragments gewonnen.

23. Das Plasmid pUC18 wird mit EcoRI und SalI geschnitten.

24. Das SalI/EcoRI Fragment, welches das in Schritt 22 erwähnte chimäre Gen enthält, wird in das mit EcoRI und SalI geschnittene Plasmid pUC18 eingespleisst.

25. Die BamHI-Erkennungsstelle in der Terminationssequenz dieses chimären Gens wird durch Schneiden mit BamHI zerstört, unter Verwendung von T4 + DNA-Polymerase ergänzt und schliesslich wieder verknüpft.

26. Das resultierende Plasmid wird mit SstII (Bethesda Research Laboratories) und HindIII geschnitten.

27. Ein 1,0 Kb umfassendes Fragment, welches den 5' gelegenen Teil des nos-Promotors und die rechte Grenzsequenz des Plasmids pTiT37 trägt, wird durch Schneiden von pBR325 (Hind23) mit HindIII und SstII isoliert.

28. Dieses 1,0 Kb umfassende HindII/SstII-Fragment wird in das Plasmid pUC18 aus Schritt 26 eingespleisst, unter Bildung von pCIB4 (Schritt 22-28, siehe Abbildung 7b).

29. Das Plasmid pCIB5, das die linke DNA Grenzsequenz enthält, wird mit AatII geschnitten, durch Behandlung mit T4 DNA Polymerase mit glatten Enden versehen und anschliessend mit EcoRI erneut geschnitten.

30. pCIB4 wird mit HindIII geschnitten, durch Behandlung mit dem Klenow Fragment der E. coli DNA Polymerase mit glatten Enden versehen und anschliessend mit EcoRI geschnitten.

31. Das nach Restriktion erhaltene Plasmid pCIB5 aus Schritt 29 wird mit dem verkürzten Plasmid pCIB4 (Schritt 30) unter Bildung von pCIB2 verknüpft, einem ColEl-Replikon, das die linke und die rechte T-DNA Grenzsequenz enthält, die ein chimäres Kanamycin
Resistenzgen und einen Polylinker flankieren (Schritt 29-31, siehe
Abbildung 7c).

32. Das Plasmid pRZ102 [Jorgenson et al., Mol. Gen. Genet; 177: 65
(1979)] wird mit BamHI verdaut und unter Verwendung von Klenow
ergänzt.

33. Es wird eine teilseise AluI-Verdauung des Plasmids pA03 [Oka,
J. Mol. Biol., 174: 217 (1981)] durchgeführt.

34. Das AluI-Verdauungsprodukt des Plasmids pA03 wird in das Plasmid
pRZ102 aus Schritt 32 eingespleisst, wobei die gewünschten Transformanten aufgrund ihrer Kanamycin-Resistenz selektiert werden
können.

35. Das resultierende Plasmid besitzt die kodierende Sequenz von
Tn903 auf einem 1,05 Kb BamHI-Fragment, das nach einer BamHI-Verdauung isoliert wird. Dieses Fragment wird anschliessend mit Klenow-
DNA-Poylymerase behandelt.

36. Das Plasmid pRK252, ein Abkömmling des Plasmids RK2, das einen
weiten Wirtsbereich aufweist, kann von Dr. Don Helinski an der
University of California in San Diego (USA) bezogen werden. Diesem
Plasmid fehlt die BglII-Seite, die in dem Stammplasmid pRK290
[Ditta et al., Proc. Natl. Acad. Sci. USA, 77: 7347 (1980)] vorliegt.
pRK252 wird mit SmaI und SalI verdaut, und mit Hilfe von Klenow
ergänzt; das aus dieser Verdauung resultierende grosse Fragment wird
isoliert.

37. Das in Schritt 35 isolierte, das Tn903 enthaltende Fragment,
wird in das grosse Fragment von pRK252 eingespleisst, unter Bildung
von pRK252Km (Schritt 32-37, siehe Abbildung 7d).

38. Das Plasmid pRK252Km wird mit EcoRI geschnitten, unter Verwendung von Klenow mit glatten Enden versehen und mit BglII-Linkern (New England Biolabs) verknüpft.

39. Das Plasmid pCIB2 wird mit EcoRV geschnitten und das kleinere Fragment, das die rechte Grenzsequenz und die nos-neo-nos-Sequenz enthält, wird isoliert. Dieses Fragment wird mit Klenow-Polymerase ergänzt und mit BglII-Linkern (New England Biolabs) verknüpft.

40. Das BglII Fragment aus Schritt 39 wird mit dem Plasmid pRK252Km aus Schritt 38, das einen Linker trägt, verknüpft, unter Bildung von pCIB10 (Schritt 38-40, siehe Abbildung 7e).

Konstruktion von pCIB10a:
41. Das Plasmid pRZ102 [Jorgensen, et al., Mol. Gen. Genet., 177: 65 (1979)] wird mit BamHI verdaut und unter Verwendung von Klenow aufgefüllt.

42. Teilverdauung des Plasmids pAO3 [Oka et al., Nature, 276: 845 (1978)].

43. Das mit Hilfe von AluI verdaute Material wird in das nach Restriktion erhaltene Plasmid pRZ102 aus Schritt 32 eingespleisst, wobei die gewünschten Transformanten aufgrund ihrer Kanamycin-Resistenz selektiert werden.

44. Das resultierende Plasmid besitzt die codierende Sequenz von Tn903 auf einem 1,05 Kb umfassenden BamHI-Fragment; dieses Fragment wird nach erfolgter BamHI-Verdauung und nach Auffüllen mit Hilfe von Klenow isoliert.

45. Das Plasmid pRK290, ein Derivat des Plasmids RK2, das einen weiten Wirtsbereich umfasst, kann von Dr. Don Helinski an der Universität von Kalifornien, San Diego (USA), bezogen werden. pRK290 wird mit SmaI und SalI verdaut, mit Klenow ergänzt und das aus dieser Verdauung resultierende grosse Fragment isoliert.

46. Das Tn903 enthaltende Fragment, welches in Schritt 35 isoliert wurde, wird in das grosse Fragment von pRK2 eingespleisst unter Bildung von pRK290Km.

47. Das Plasmid aus Schritt 46 wird mit BglII verdaut, mit Hilfe von Klenow ergänzt und unter Zerstörung seiner BglII-Stelle wieder verknüpft. Es entsteht das Plasmid pKR290Km (Schritt 41-47, siehe Abbildung 7f).

48. Das Plasmid pRK290Km wird mit EcoRI geschnitten, unter Verwendung von Klenow mit glatten Enden versehen und mit BglII-Linkern (New England Biolabs) ausgestattet.

49. Das Plasmid pCIB2 wird mit EcoRV geschnitten und ebenfalls mit BglII-Linkern (New England Biolabs) versehen.

50. Das mit BglII-Linkern ausgestattete Plasmid pCIB2 aus Schritt 39 wird in den Vektor aus Schritt 47 eingespleisst unter Bildung von pCIB10a (Schritt 48-50, siehe Abbildung 7g).

Die unter Punkt 41-50 beschriebenen Verfahrensschritte können in gleicher Weise unter Verwendung des Plasmids pRK252 anstelle von pRK290 durchgeführt werden.

Beispiel VI: Konstruktion von pCIB23 und pCIB24, Vektoren, die das GST-Enzym auf die Chloroplasten der transformierten Pflanzen ausrichten

Das Plasmid pSRS2.1, das die 5' Sequenz der kleinen Untereinheit (SSU) der Ribulose-bis-phosphat-carboxylase (RuPBC) von Sojabohnen enthält [Berry-Lowe et al., J. Mol. Appl. Genet., 1: 483-498 (1982)] ist bei Dr. Richard Meagher vom Department of Genetics der University of Georgia in Athens, Georgia 30602 (USA) erhältlich.

Dieses Plasmid wird mit EcoRI verdaut. Das 2,1 Kb umfassende Fragment, das die SSU 5' Region aus Sojabohnen enthält, wird von einem Agarose-Gel isoliert. Das 2,1 Kb EcoRI-Fragment wird anschliessend mit DdeI verdaut und ein 471 Bp umfassendes DdeI-Fragment wird isoliert. Dieses Fragment enthält das Transit-Peptid und einen Teil des zweiten Exon.

Das 471 Bb umfassende DdeI-Fragment wird mit Klenow (New England Biolabs DNA Polymerase) behandelt. Ein mit Kinase behandelter BglII-Linker [d(CAGATCTG) New England Biolabs] wird mit diesem Fragment verknüpft. Dieses BglII-Fragment wird anschliessend mit TaqI verdaut und die resultierenden TaqI Fragmente werden mit Klenow (Bethesda Research Labs DNA Polymerase) behandelt. Die resultierenden glatt endenden Fragmente werden mit BamHI-Linkern verknüpft, die zuvor mit Kinase behandelt worden sind [d(CGCGGATCCGCG) New England Biolabs] und anschliessend gereinigt. Diese BamHI-Fragmente werden dann mit BglII und BamHI verdaut. Ein BamHI/BglII-Fragment von annähernd 400 Bp, das die SSU 5' Region enthält, wird gereinigt.

Das Plasmid pCIB710 wird mit BamHI geschnitten und anschliessend mit alkalischer Phosphatase aus Kälberdarm behandelt. Das 400 Bp umfassende BamHI/BglII-Fragment (siehe oben) wird in dieses Plasmid (pCIB710) eingespleisst. Das Verknüpfungsprodukt wird in E.coli HB101 übertragen und die Transformanten werden auf Ampillicin selektiert.

Man findet auf diese Weise Transformanten, die das BamHI/BglII-Fragment in beiden Orientierungen tragen. Im Plasmid pSCR2 befindet sich die 5' Region des Transit-Polypeptids unmittelbar neben dem 35S Promotor. Demgegenüber liegt das BamHI/BglII-Fragment im Plasmid pSCR1 in der entgegengesetzten Orientierung vor.

Das Plasmid pCIB12 wird mit BamHI verdaut und das 708 Bp umfassende BamHI-Fragment, das das GST-Gen trägt, wird isoliert.

Das Plasmid pSCR2 wird ebenfalls mit BamHI geschnitten und anschliessend mit alkalischer Phosphatase aus Kälberdarm behandelt. Das 708 Bp umfassende BamHI-Fragment aus dem Plasmid pCIB12 wird dann in das mit BamHI behandelte Plasmid pSCR2 eingespleisst. Das Verknüpfungsprodukt wird in HB101 überführt und die Transformanten auf Ampillicin selektiert.

Man findet Transformanten, die das GST-Gen in beiden Orientierungen im Hinblick auf die Kontroll-Region am 5' Ende aufweisen. Der Klon pCIB22 besitzt das GST-Gen in einer für die Transkription, ausgehend vom CaMV-Promotor, geeigneten Orientierung. Im Klon pCIB21 dagegen weist das GST-Gen die entgegengesetzte Orientierung auf. pCIB22 wird mit XbaI/EcoRI verdaut. Das Fragment, welches das chimäre Gen trägt, wird aus einem Gel isoliert und gereinigt.

Das Plasmid pCIB10 wird ebenfalls mit XbaI und EcoRI verdaut. Das XbaI/EcoRI Fragment, welches das chimäre Gen trägt, wird in das zuvor verdaute Plasmid pCIB10 eingespleisst und die Transformanten werden anhand ihrer Kanamycin-Resistenz selektiert. Bei dem resultierenden Plasmid pCIB24 handelt es sich um ein Plasmid mit weitem Wirtsbereich, welches das chimäre Gen in unmittelbarer Verbindung mit einer Chloroplasten Transit-Peptid-Sequenz enthält.

Das Plasmid pCIB23 wird, ausgehend von dem Klon pCIB21 anstelle von pCIB22 und unter Verwendung ähnlicher manipulatorischer Schritte, konstruiert. Dieses Plasmid enthält das GST-Gen in der entgegengesetzten Orientierung im Vergleich zu pCIB24.

Diese Plasmide werden in <u>Agrobacterium</u> Stämme übertragen und zwar in ähnlicher Weise wie dies zuvor für pCIB13 und pCIB14 beschrieben wurde.

<u>Beispiel VII</u>: <u>Konstruktion des Plasmids pCIB542, eines Agropin Vir</u>
<u>Helfer-Plasmids, das ein Spectinomycin-Resistenz-Gen</u>
<u>im Bereich der T-DNA trägt</u>

Das Ti Plasmid pTiBo542 |Sciaky, D., Montoya, A.L. & Chilton, M-D,
<u>Plasmid</u>, <u>1</u>: 238-253 (1978)| ist von besonderem Interesse, da
Agrobakterien, die dieses Plasmid enthalten, in der Lage sind, die
agronomisch bedeutsamen Leguminosen, Alfalfa und Sojabohnen zu
infizieren |Hood, E.E., <u>et al.</u>, <u>Bio/Technology</u>, <u>2</u>: 702-708 (1984)|.

Die Konstruktion eines pTiBo542-Derivats, das im Bereich seiner
T-DNA eine Deletion aufweist, ist bei Hood, Elizabeth E., (1985)
Ph.D. thesis; Washington University, St. Louis, Mo. (USA) beschrieben. Bei dieser Konstruktion mit der Bezeichnung EHA101, ist die
T-DNA durch ein Kanamycin-Resistenz-Gen ersetzt. Das Ausgangsplasmid
von EHA101, A281, ist bei ATCC unter der ATCC-Nr. 53487 hinterlegt.

Ausgehend von EHA101 wird ein Derivat konstruiert, bei dem das
Kanamycin-Resistenz-Gen durch ein Spectinomycin-Resistenz-Gen
ersetzt ist. Das Plasmid p pi delta 307 |E. Hood, Washington
University, Ph. D. thesis (1985)| besitzt eine 1,7 Kb umfassende
Region, die homolog ist zur linken Seite von Bam a des Plasmids
pTiBo542 |Hood, <u>et al.</u>, (1984)], sowie eine 8 Kb umfassende Region,
die homolog ist zur rechten Seite von Bam2a von pTiBo542, getrennt
durch eine einzelne EcoRI-Stelle. Das Plasmid pMON30,
ATCC Nr. 67113, enthält das Spectinomycin/Streptomycin-Resistenz-Gen
(spc/str) von Tn7 [Hollingshead, S. and Vapnek, D., <u>Plasmid</u>, <u>13</u>:
17-30 (1985)]. Das Plasmid pMON30 wird mit EcoRI verdaut; das 5,5 Kb
umfassende Fragment, das die spc/str-Gene enthält, wird aus einem
Agarose-Gel isoliert und in das durch EcoRI verkürzte Plasmid
p pi delta 307 eingespleisst. Das gewünschte Rekombinationsprodukt
wird in Form eines Spectinomycin-resistenten (50 µg/ml) und eines
Tetrazyklin-resistenten (10 µg/ml) Transformanten selektiert. Dieses
Plasmid wird in den <u>Agrobacterium</u> Stamm A136/EHA101 eingeschleust
und anhand seines Streptomycinresistenz-, Tetrazyklinresistenz- und
Kanamycinresistenz-Phenotyps selektiert. Merodiploide Zellen
("homogenotes") |Matzke, A.J.M. & Chilton, M-D, <u>J. Mol. Appl.</u>

Genet., 1: 39-49 (1981)] mit dem EHA101-Plasmid und dem Spectino-
mycin-Plasmid werden nach der Einschleusung des Austreibungsplasmids
R751-pMG2 [Jacoby, G. et al., J. Bacteriol., 127: 1278-1285 (1976)]
und Selektion auf Gentamycin (50 µg/ml) und Spectinomycin ausgelesen.

Die bevorzugte merodiploide Zelle zeichnet sich durch einen Gentamycin-resistenten, Spectinomycin-resistenten, Tetrazyklin-sensitiven
und Kanamycin-sensitiven Phenotyp aus. Die Struktur des resultierenden Plasmids wird mit Hilfe von "Southern blot" Untersuchungen
bestätigt.

Beispiel VIII: Test von Pflanzen auf Atrazintoleranz
Aufgrund zahlreicher Untersuchungen, wie
1) der Fluoreszenzinduktion; und
2) der Wachstumsfähigkeit von Sämlingen bei Atrazin-Konzentrationen,
die für Kontroll- oder Wild-Typ-Pflanzen toxisch sind
konnte gezeigt werden, dass regenerierte Tabakpflanzen, die die
GST-Genkonstruktion pCIB14 tragen, tolerant sind gegenüber Atrazin.

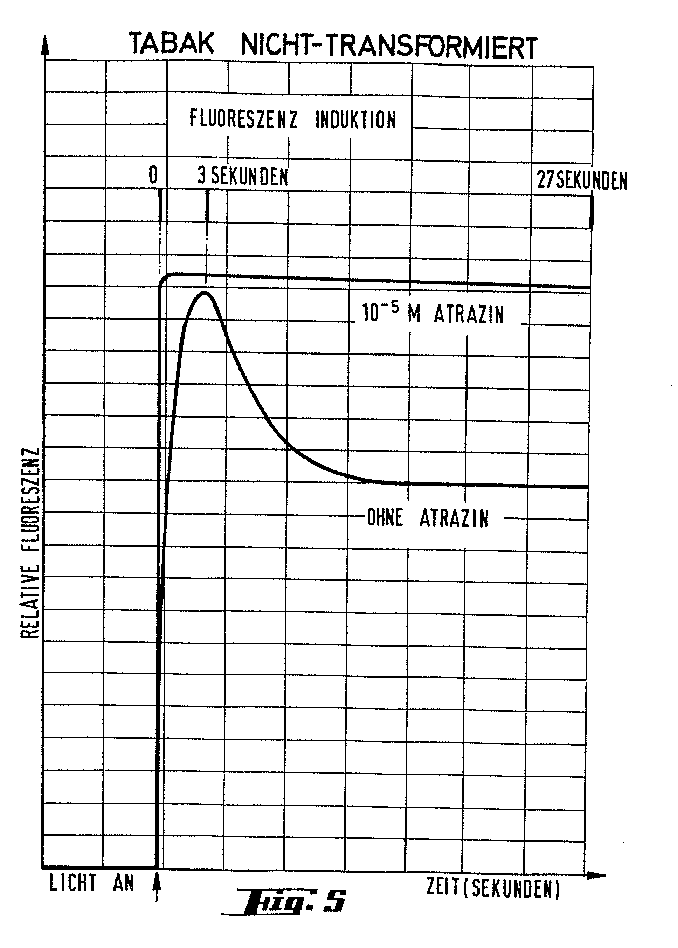

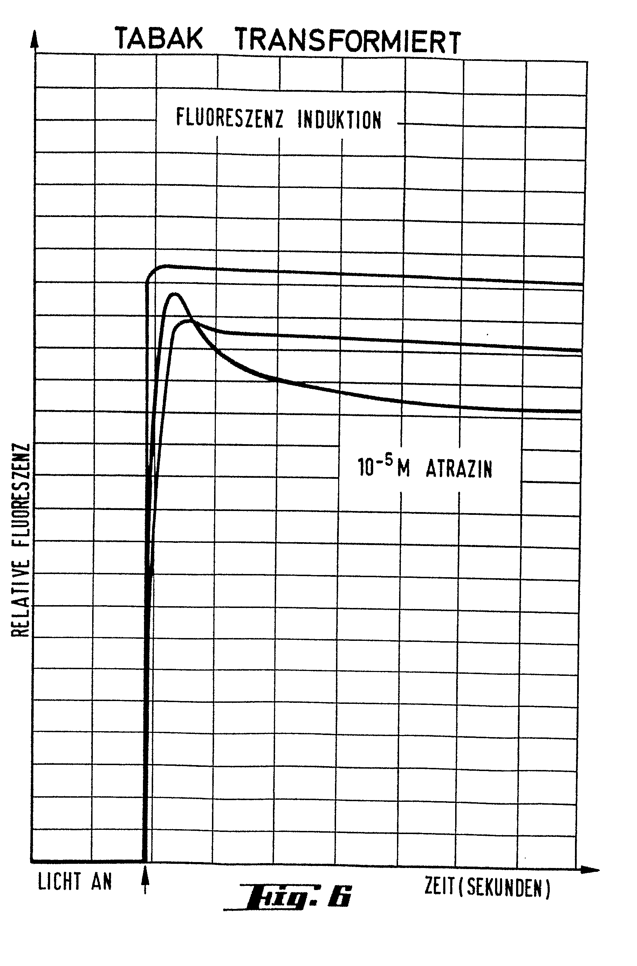

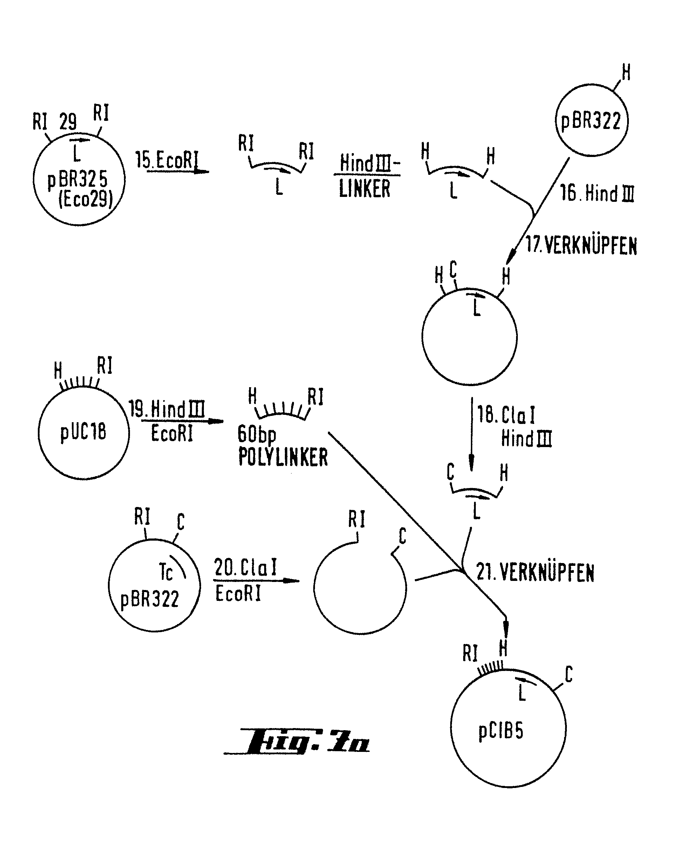

Die Fluoreszenzinduktion gibt Auskunft über den Zustand des photochemischen Apparates der Pflanze [Voss et al., Weed Science, 32:
675-680 (1984)]. Im Verlauf dieses Assays wird Blattgewebe mit Licht
einer bestimmten Wellenlänge bestrahlt und die resultierende
Fluoreszenz bei einer zweiten Wellenlänge registriert. Abbildung 5
zeigt ein Fluoreszenzinduktionsmuster, das typisch ist für ein
ausgestanztes (nicht transformiertes) Tabakblatt, das mit Pufferlösung infiltriert ist, die über den abgeschnittenen Petiolus
(Blattstiel) aufgenommen wird (untere Kurve). Man erkennt zunächst
einen starken Anstieg der Fluoreszenz, wenn das Licht eingeschaltet
wird, der dann einen Peak erreicht, gefolgt von einer allmählichen
Abnahme des Signals mit der Zeit.

Dieses Muster macht deutlich, dass die Chloroplasten durch das
einfallende Licht bei einer Wellenlänge, die charakteristisch ist
für das System, zur Fluoreszenz angeregt werden. An diesem Punkt wird Energie aus dem Photosystem in Form von Elektronen herausgeschleust, die über die Elektronentransportwege von Photosystem II und I abfliessen --- dies wird wiedergegeben durch den allmählichen Abfall der Kurve für das Fluoreszenzsignal.

Wenn aber das Blatt mit einer $10^{-5}$ M Atrazinlösung infiltriert ist, ergibt sich ein Bild, wie es in der oberen Kurve der Abbildung 5 wiedergegeben ist. In diesem Fall wird ebenfalls zunächst die Lichtenergie absorbiert, wodurch die Chloroplasten zur Fluoreszenz angeregt werden. Es findet aber kein Energieabfluss statt, da der Elektronenfluss auf der Stufe der Chinonbindung des Photosystem II blockiert ist. Es sieht aus, als wäre das Photosystem im angeregten Zustand erstarrt. Daher lässt sich nach der anfänglich starken Zunahme der Fluoreszenz keinerlei Abfall erkennen.

Werden diese Messungen bei gemäss der vorliegenden Erfindung genetisch manipulierten Tabakpflanzen in Gegenwart von $10^{-5}$ M Atrazin durchgeführt, so zeigen alle Kontrollpflanzen die gleichen Fluoreszenz-Induktionsmuster wie die nicht transgenen Tabakpflanzen.

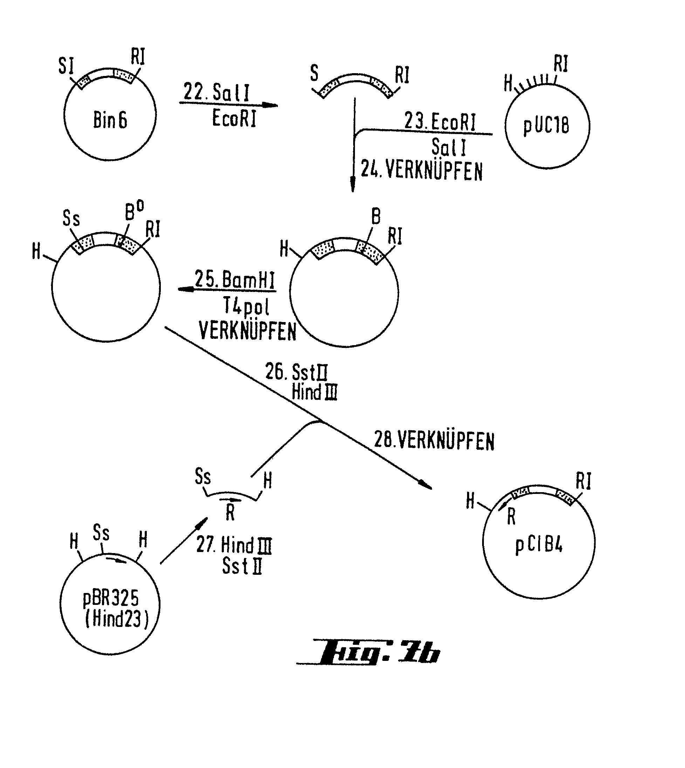

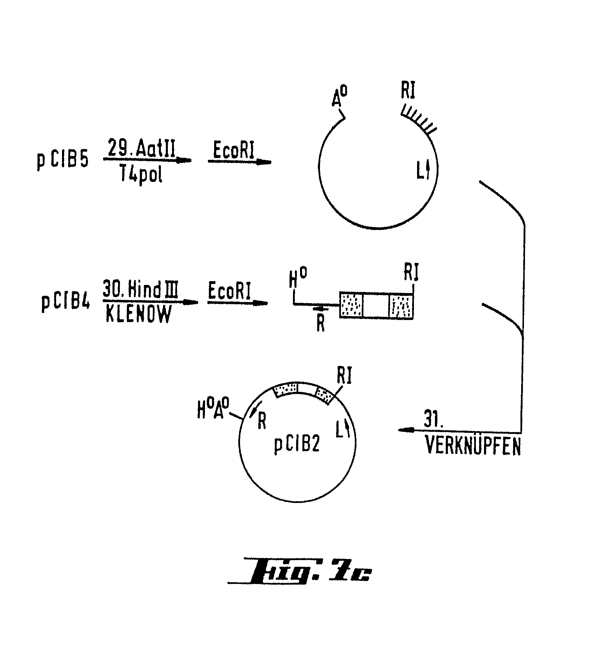

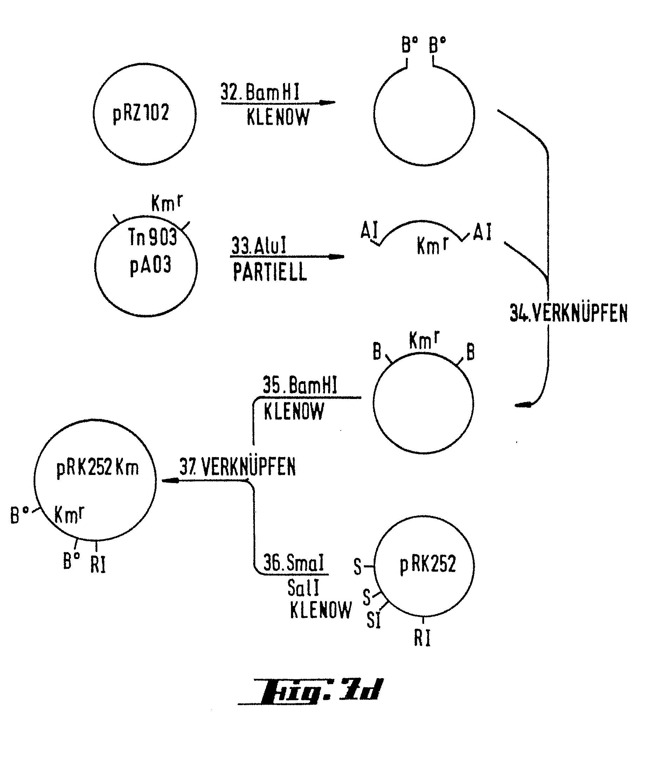

Werden die Messungen dagegen bei den Versuchspflanzen vorgenommen, so lassen sich drei Klassen von Fluoreszenz-Induktionsmustern unterscheiden (Abbildung 6).

Einige der transgenen Pflanzen zeigen keinerlei Anhaltspunkte für eine Atrazin-Detoxifikation (die oberste Kurve), einige zeigen eine mittlere Detoxifikation (mittlere Kurve) und einige lassen eine signifikante (untere Kurve) Atrazin-Detoxifikation erkennen, was aufgrund des normalen Elektronenabflusses durch das Photosystem offensichtlich wird. Von 27 Pflanzen, die auf diese Weise charakterisiert wurden, zeigten 5 eindeutige Hinweise auf die Fähigkeit zur Detoxifikation von Atrazin.

Beispiel IX:

Agrobacterium-Infektion pflanzlichen Materials

Die verschiedenen Genotypen von Agrobacterium tumefaciens werden auf einem AB Minimal-Medium [Watson, B. et al., J. Bacteriol., 123: 255-264 (1975)], dem Mannitol zugesetzt ist, 48 Stunden bei einer Temperatur von 28°C kultiviert. Die Bakterien werden pelletiert, in MSBN-Medium mit einer zweifachen Verdünnung resuspendiert und für drei Stunden bei einer Temperatur von 25°C inkubiert. Für die Herstellung des MSBN-Mediums wird eine vorgefertigte pulverförmige Mixtur verwendet, die von KC Biologicals bezogen werden kann und die die Makro- und Mikroelemente des Murashige und Skoog Mediums in Form ihrer Salze enthält. Die Endkonzentration der Bestandteile des MSBN-Mediums entspricht derjenigen des Mediums nach Murashige und Skoog. Zusätzlich enthält das MSBN-Medium (Endkonzentrationen): Benzyladenin (1 mg/l); Naphthylessigsäure (0,1 mg/l); Myo-Inositol (1 mg/l); Nicotinsäure (1 mg/l); Pyridoxin (1 mg/l); Thiamin HCl (10 mg); und Sucrose (30 mg/l)]. Der pH wird auf einen Wert von pH 5,7 bis 5,8 eingestellt.

Man lässt Blattscheiben von in vitro kultivierten Nicotiana tabacum cv. petite Havana SR1 Pflanzen 10 Minuten auf einer Bakteriensuspension aufschwimmen in einer Modifikation einer Methode von Horsch, R. et al., Science, 227: 1229-1231 (1985). Die Blattscheiben werden dann auf Filterpapier auf ein MSBN-Medium ohne Antibiotika übertragen. Nach 48 Stunden werden die Blattstückchen in flüssiges MSBN-Medium eingetaucht, das 500 mg/l Carbenicillin enthält, und anschliessend auf ein festes Selektionsmedium übertragen, das 100 mg/l Kanamycin und 500 mg/l Carbenicillin enthält.

Pflanzenreifung und Selbstbestäubung

Schösslinge, die sich auf dem Selektionsmedium aus den Kalli entwickeln, werden entfernt und auf OMS-Medium mit 100 mg/l Kanamycin und 250 mg/l Carbenicillin übertragen. Man lässt dann die Entwicklung für drei weitere Wochen fortschreiten. Anschliessend werden die Pflänzchen in Erde ausgepflanzt und ins Gewächshaus überführt. Die Blütenbildung beginnt 4 bis 8 Wochen nach dem

Verbringen der Pflanzen ins Gewächshaus. Zu dem Zeitpunkt, da sich die Blüte öffnet und ihre Antheren sich voneinander trennen, werden diese mit Hilfe einer Pinzette entfernt und zur Selbstbestäubung jeder einzelnen Blüte auf die Narben aufgerieben. Die Samenkapseln reifen innerhalb von 40 Tagen.

## Testung der Samen-Nachkommenschaft von Kontrollpflanzen, transgenen Kontrollpflanzen und transgenen Versuchspflanzen

Die Samen werden zunächst unter keimfreien Bedingungen aus der reifen Samenkapsel entfernt und in sterilen Petrischalen aufbewahrt. Die Samen werden anschliessend auf ein mittelweiches Samenkeimungsmedium (SKM) überführt, das zusammengesetzt ist aus sämtlichen Makro- und Mikroelementen in Form ihrer Salze und in einer Konzentration, entsprechend dem Medium nach Murashige & Skoog (KC Biologicals), aus 1 mg/l Thiaminhydrochlorid sowie 0,6 % gereinigtem Agar (Difco®). Dem Medium wird Atrazin in Analysequalität, in einer Konzentration von $10^{-7}$M, $3 \times 10^{-7}$M, $5 \times 10^{-7}$M, $8 \times 10^{-7}$M und $10^{-6}$M zugesetzt.

Wachstum und Ueberlebensrate der Sämlinge wird bei den genannten Konzentrationen sowie bei einem Atrazinlevel von Null, über einen Zeitraum von 14 Tagen bestimmt; die Ergebnisse sind in Tabelle 1, unten, wiedergegeben. Alle Kontrollpflanzen und transgenen Kontrollpflanzen keimen zwar aus, erreichen aber in ihrem Wachstum bei Atrazinkonzentrationen von $5 \times 10^{-7}$M oder bei höheren Konzentra-tionen lediglich das Kotyledonenstadium. Unter den Sämlingen, die sich aus Samen entwickeln, der aus der Selbstbestäubung von pCIB14-Pflanzen hervorgegangen ist, bleiben bei einer Atrazinkonzen-tration von $5 \times 10^{-7}$M annähernd 75 % grün und bilden Primärblätter aus, während diese Sämlinge bei Atrazinkonzentrationen von $8 \times 10^{-7}$M oder höheren Konzentrationen nur noch Kotyledonen ausbilden bevor sie ausbleichen und absterben.

Obwohl die toleranten Sämlinge auf Atrazin-haltigem Medium nicht das gleiche gute Wachstum wie auf Atrazin-freiem Medium zeigen, können sie dennoch sehr leicht von den sensitiven Kontrollen auf dem gleichen Medium unterschieden werden.

| Genotyp der Sämlinge | Atrazin-Konzentrationen [M] | | | | |
|---|---|---|---|---|---|
| | $10^{-7}$M | $3 \times 10^{-7}$M | $5 \times 10^{-7}$M | $8 \times 10^{-7}$M | $10^{-6}$M |
| Kontrolle | + | + | o | o | o |
| LBA 4404 | + | + | o | o | o |
| Bin 6 | + | + | o | o | o |
| pCIB13 | + | + | o | o | o |
| pCIB14 | + | + | + | o | o |

Die Tabelle macht den Unterschied im Wachstum und Ueberleben zwischen den Nachkommen von transgenen pCIB14-Pflanzen und den Kontrollen deutlich. Positives Wachstum ist durch "+" gekennzeichnet, negatives Wachstum durch ein "o".

Obwohl die vorliegende Erfindung zuvor anhand von Abbildungen und Beispielen zum Zwecke der Anschaulichkeit und Verständlichkeit in verschiedenen Einzelheiten beschrieben worden ist, ist dennoch klar, dass im Rahmen dieser Erfindung gewisse Veränderungen und Modifikationen durchführbar sind, deren Grenzen einzig und allein durch den Umfang der beigefügten Ansprüche vorgegeben sind.

Im Rahmen der vorliegenden Erfindung bilden nicht nur jene DNA-Moleküle, die mit konkreter Nucleotid-Sequenz angegeben sind und für ein Glutathion-S-Transferase-Polypeptid kodieren, einen Bestandteil der Erfindung, sondern gleichermassen deren Varianten oder Mutanten, die für Polypeptide mit Glutathion-S-Transferase-Aktivität kodieren.

Patentansprüche

1. Ein rekombinantes DNA-Molekül, dadurch gekennzeichnet, dass es befähigt ist eine Herbizid-Toleranz, die auf der Detoxifikation des Herbizids beruht, auf eine Pflanze zu übertragen.

2. Ein rekombinantes DNA-Molekül gemäss Anspruch 1, dadurch gekennzeichnet, dass es sich bei besagten Herbiziden um ein Chloracetamid, einen Sulfonylharnstoff, ein Triazin, einen Diphenylether, ein Imidazolinon oder um ein Thiocarbamat handelt.

3. Ein rekombinantes DNA-Molekül, dadurch gekennzeichnet, dass es eine Gen-Sequenz aufweist, die für ein Glutathion-S-Transferase-Polypeptid kodiert.

4. Ein rekombinantes DNA-Molekül gemäss Anspruch 1, enthaltend eine Gen-Sequenz, die in Pflanzenzellen exprimiert wird.

5. Ein rekombinantes DNA-Molekül gemäss Anspruch 4, dadurch gekennzeichnet, dass es sich bei besagten Pflanzenzellen um Zellen von Tabak, Sojabohnen oder Baumwolle handelt.

6. Ein rekombinantes DNA-Molekül gemäss Anspruch 4, dadurch gekennzeichnet, dass besagte pflanzliche Zelle ein Bestandteil einer ganzen Pflanze ist.

7. Ein rekombinantes DNA-Molekül gemäss Anspruch 3, dadurch gekennzeichnet, dass die für ein Glutathion-S-Transferase-Polypeptid kodierende Gen-Sequenz von einem Säuger stammt.

8. Ein rekombinantes DNA-Molekül gemäss Anspruch 7, dadurch gekennzeichnet, dass die für ein Glutathion-S-Transferase-Polypeptid kodierende Gen-Sequenz von einer Ratte stammt.

9. Ein rekombinantes DNA-Molekül gemäss Anspruch 8, dadurch gekennzeichnet, dass die für ein Glutathion-S-Transferase-Polypeptid der
Ratte kodierende Gen-Sequenz die folgende Nucleotid-Sequenz aufweist:

```
                                          40        50        60
                                   ATGCCTATGATACTGGGATACTGG
                                    M  P  M  I  L  G  Y  W
        70        80        90       100       110       120
AACGTCCGCGGGCTGACACACCCGATCCGCCTGCTCCTGGAATACACAGACTCAAGCTAT
 N  V  R  G  L  T  H  P  I  R  L  L  L  E  Y  T  D  S  S  Y
       130       140       150       160       170       180
GAGGAGAAGAGATACGCCATGGGCGACGCTCCCGACTATGACAGAAGCCAGTGGCTGAAT
 E  E  K  R  Y  A  M  G  D  A  P  D  Y  D  R  S  Q  W  L  N
       190       200       210       220       230       240
GAGAAGTTCAAACTGGGCCTGGACTTCCCCAATCTGCCCTACTTAATTGATGGATCGCGC
 E  K  F  K  L  G  L  D  F  P  N  L  P  Y  L  I  D  G  S  R
       250       260       270       280       290       300
AAGATTACCCAGAGCAATGCCATAATGCGCTACCTTGCCCGCAAGCACCACCTGTGTGGA
 K  I  T  Q  S  N  A  I  M  R  Y  L  A  R  K  H  H  L  C  G
       310       320       330       340       350       360
GAGACAGAGGAGGAGCGGATTCGTGCAGACATTGTGGAGAACCAGGTCATGGACAACCGC
 E  T  E  E  E  R  I  R  A  D  I  V  E  N  Q  V  M  D  N  R
       370       380       390       400       410       420
ATGCAGCTCATCATGCTTTGTTACAACCCCGACTTTGAGAAGCAGAAGCCAGAGTTCTTG
 M  Q  L  I  M  L  C  Y  N  P  D  F  E  K  Q  K  P  E  F  L
       430       440       450       460       470       480
AAGACCATCCCTGAGAAGATGAAGCTCTACTCTGAGTTCCTGGGCAAGCGACCATGGTTT
 K  T  I  P  E  K  M  K  L  Y  S  E  F  L  G  K  R  P  W  F
       490       500       510       520       530       540
GCAGGGGACAAGGTCACCTATGTGGATTTCCTTGCTTATGACATTCTTGACCAGTACCAC
 A  G  D  K  V  T  Y  V  D  F  L  A  Y  D  I  L  D  Q  Y  H
       550       560       570       580       590       600
ATTTTTGAGCCCAAGTGCCTGGACGCCTTCCCAAACCTGAAGGACTTCCTGGCCCGCTTC
 I  F  E  P  K  C  L  D  A  F  P  N  L  K  D  F  L  A  R  F
       610       620       630       640       650       660
GAGGGCCTGAAGAAGATCTCTGCCTACATGAAGAGCAGCCGCTACCTCTCAACACCTATA
 E  G  L  K  K  I  S  A  Y  M  K  S  S  R  Y  L  S  T  P  I
       670       680       690       700       710       720
TTTTCGAAGTTGGCCCAATGGAGTAACAAGTAG
 F  S  K  L  A  Q  W  S  N  K
```

sowie deren Varianten oder Mutanten, die für Polypeptide mit
Glutathion-S-Transferase-Aktivität kodieren.

10. Ein rekombinantes DNA-Molekül gemäss Anspruch 8, dadurch gekennzeichnet, dass die für eine Glutathion-S-Transferase der Ratte kodierende Gen-Sequenz die folgende Nucleotid-Sequenz aufweist:

```
                        1 10                        13 0
          TAC AGC ATG GGG GAT GCT CCC GAC TAT GAC AGA AGC CAG
          Tyr Ser Met Gly Asp Ala Pro Asp Tyr Asp Arg Ser Gln

                         150                        1 70
      TGG CTG AGT GAG AAG TTC AAA CTG GGC CTG GAC TTC CCC AAT CTG
      Trp Leu Ser Glu Lys Phe Lys Leu Gly Leu Asp Phe Pro Asn Leu

                         19 0                        210
      CCC TAC TTA ATT GAT GGG TCA CAC AAG ATC ACC CAG AGC AAT GCC
      Pro Tyr Leu Ile Asp Gly Ser His Lys Ile Thr Gln Ser Asn Ala

       2 30                        25 0                        270
      ATC CTG CGC TAC CTT GGC CGG AAG CAC AAC CTT TGT GGG GAG ACA
      Ile Leu Arg Tyr Leu Gly Arg Lys His Asn Leu Cys Gly Glu Thr

                         2 90                        31 0
      GAG GAG GAG AGG ATT CGT GTG GAC GTT TTG GAG AAC CAG GCT ATG
      Glu Glu Glu Arg Ile Arg Val Asp Val Leu Glu Asn Gln Ala Met

                         330                        3 50
      GAC ACC CGC CTA CAG TTG GCC ATG GTC TGC TAC AGC CCT GAC TTT
      Asp Thr Arg Leu Gln Leu Ala Met Val Cys Tyr Ser Pro Asp Phe

                  37 0                        390
      GAG AGA AAG AAG CCA GAG TAC TTA GAG GGT CTC CCT GAG AAG ATG
      Glu Arg Lys Lys Pro Glu Tyr Leu Glu Gly Leu Pro Gly Lys Met

       4 10                        43 0                        450
      AAG CTT TAC TCC GAA TTC CTG GGC AAG CAG CCA TGG TTT GCA GGG
      Lys Leu Tyr Ser Glu Phe Leu Gly Lys Gln Pro Trp Phe Ala Gly

                         4 70                        49 0
      AAC AAG ATT ACG TAT GTG GAT TTT CTT GTT TAC GAT GTC CTT GAT
      Asn Lys Ile Thr Tyr Val Asp Phe Leu Val Tyr Asp Val Leu Asp

                         510                        5 30
      CAA CAC CGT ATA TTT GAA CCC AAG TGC CTG GAC GCC TTC CCA AAC
      Gln His Arg Ile Phe Glu Pro Lys Cys Leu Asp Ala Phe Pro Asn

                  55 0                        570
      CTG AAG GAC TTC GTG GCT CGG TTT GAG GGC CTG AAG AAG ATA TCT
      Leu Lys Asp Phe Val Ala Arg Phe Glu Gly Leu Lys Lys Ile Ser

       5 90                        61 0                        630
      GAC TAC ATG AAG AGC GGC CGC TTC CTC TCC AAG CCA ATC TTT GCA
      Asp Tyr Met Lys Ser Gly Arg Phe Leu Ser Lys Pro Ile Phe Ala

                         6 50
      AAG ATG GCC TTT TGG AAC CCA AAG TAG
      Lys Met Ala Phe Trp Asn Pro Lys End
```

sowie deren Varianten oder Mutanten, die für Polypeptide mit
Glutathion-S-Transferase-Aktivität kodieren.

11. Ein rekombinantes DNA-Molekül gemäss Anspruch 8, dadurch
gekennzeichnet, dass die für ein Glutathion-S-Transferase-Polypeptid
der Ratte kodierende Gen-Sequenz die folgende Nucleotid-Sequenz
aufweist:

```
          1 0                          30
ATG CCT ATG ACA CTG GGT TAC TGG GAC ATC CGT GGG CTG GCT CAC
Met Pro Met Thr Leu Gly Tyr Trp Asp Ile Arg Gly Leu Ala His
      50                          7 0                          90
GCC ATT CGC CTG TTC CTG GAG TAT ACA GAC ACA AGC TAT GAG GAC
Ala Ile Arg Leu Phe Leu Glu Tyr Thr Asp Thr Ser Tyr Glu Asp
                          1 10                    13 0
AAG AAG TAC AGC ATG GGG GAT GCT CCC GAC TAT GAC AGA AGC CAG
Lys Lys Tyr Ser Met Gly Asp Ala Pro Asp Tyr Asp Arg Ser Gln
              150                          1 70
TGG CTG AGT GAG AAG TTC AAA CTG GGC CTG GAC TTC CCC AAT CTG
Trp Leu Ser Glu Lys Phe Lys Leu Gly Leu Asp Phe Pro Asn Leu
          19 0                          210
CCC TAC TTA ATT GAT GGG TCA CAC AAG ATC ACC CAG AGC AAT GCC
Pro Tyr Leu Ile Asp Gly Ser His Lys Ile Thr Gln Ser Asn Ala
      2 30                          25 0                          270
ATC CTG CGC TAC CTT GGC CGG AAG CAC AAC CTT TGT GGG GAG ACA
Ile Leu Arg Tyr Leu Gly Arg Lys His Asn Leu Cys Gly Glu Thr
                          2 90                    31 0
GAG GAG GAG AGG ATT CGT GTG GAC GTT TTG GAG AAC CAG GCT ATG
Glu Glu Glu Arg Ile Arg Val Asp Val Leu Glu Asn Gln Ala Met
              330                          3 50
GAC ACC CGC CTA CAG TTG GCC ATG GTC TGC TAC AGC CCT GAC TTT
Asp Thr Arg Leu Gln Leu Ala Met Val Cys Tyr Ser Pro Asp Phe
          37 0                          390
GAG AGA AAG AAG CCA GAG TAC TTA GAG GGT CTC CCT GAG AAG ATG
Glu Arg Lys Lys Pro Glu Tyr Leu Glu Gly Leu Pro Glu Lys Met
      4 10                          43 0                          450
AAG CTT TAC TCC GAA TTC CTG GGC AAG CAG CCA TGG TTT GCA GGG
Lys Leu Tyr Ser Glu Phe Leu Gly Lys Gln Pro Trp Phe Ala Gly
                          4 70                    49 0
AAC AAG ATT ACG TAT GTG GAT TTT CTT GTT TAC GAT GTC CTT GAT
Asn Lys Ile Thr Tyr Val Asp Phe Leu Val Tyr Asp Val Leu Asp
              510                          5 30
CAA CAC CGT ATA TTT GAA CCC AAG TGC CTG GAC GCC TTC CCA AAC
Gln His Arg Ile Phe Glu Pro Lys Cys Leu Asp Ala Phe Pro Asn
```

```
      55 0                            570
CTG AAG GAC TTC GTG GCT CGG TTT GAG GGC CTG AAG AAG ATA TCT
Leu Lys Asp Phe Val Ala Arg Phe Glu Gly Leu Lys Lys Ile Ser

    5 90                            61 0                    630
GAC TAC ATG AAG AGC GGC CGC TTC CTC TCC AAG CCA ATC TTT GCA
Asp Tyr Met Lys Ser Gly Arg Phe Leu Ser Lys Pro Ile Phe Ala

                    6 50
AAG ATG GCC TTT TGG AAC CCA AAG TAG
Lys Met Ala Phe Trp Asn Pro Lys End
```

sowie deren Varianten oder Mutanten, die für Polypeptide mit Glutathion-S-Transferase-Aktivität kodieren.


12. Ein rekombinantes DNA-Molekül gemäss Anspruch 8, mit folgender

Nucleotid-Sequenz:

```
              10                         30
  A GAC CCC AGC ACC ATG CCC ATG ACA CTG GGT TAC TGG GAC ATC
                  Met Pro Met Thr Leu Gly Tyr Trp Asp Ile

      5 0                         70
CGT GGG CTA GCG CAT GCC ATC CGC CTG CTC CTG GAA TAC ACA GAC
Arg Gly Leu Ala His Ala Ile Arg Leu Leu Leu Glu Tyr Thr Asp

90                         11 0                    130
TCG AGC TAT GAG GAG AAG AGA TAC ACC ATG GGA GAC GCT CCC GAC
Ser Ser Tyr Glu Glu Lys Arg Tyr Thr Met Gly Asp Ala Pro Asp

                  1 50                         17 0
TTT GAC AGA AGC CAG TGG CTG AAT GAG AAG TTC AAA CTG GGC CTG
Phe Asp Arg Ser Gln Trp Leu Asn Glu Lys Phe Lys Leu Gly Leu

              190                         2 10
GAC TTC CCC AAT CTG CCC TAC TTA ATT GAT GGA TCA CAC AAG ATC
Asp Phe Pro Asn Leu Pro Tyr Leu Ile Asp Gly Ser His Lys Ile

        23 0                         250                    2
ACC CAG AGC AAT GCC ATC CTG CGC TAT CTT GGC CGC AAG CAC AAC
Thr Gln Ser Asn Ala Ile Leu Arg Tyr Leu Gly Arg Lys His Asn

70            -            29 0                    310
CTG TGT GGG GAG ACA GAA GAG GAG AGG ATT CGT GTG GAC ATT CTG
Leu Cys Gly Glu Thr Glu Glu Glu Arg Ile Arg Val Asp Ile Leu

              3 30                         35 0
GAG AAT CAG CTC ATG GAC AAC CGC ATG GTG CTG GCG AGA CTT TGC
Glu Asn Gln Leu Met Asp Asn Arg Met Val Leu Ala Arg Leu Cys

              370                         3 90
TAT AAC CCT GAC TTT GAG AAG CTG AAG CCA GGG TAC CTG GAG CAA
Tyr Asn Pro Asp Phe Glu Lys Leu Lys Pro Gly Tyr Leu Glu Gln

        41 0                         430                    4
CTG CCT GGA ATG ATG CGG CTT TAC TCC GAG TTC CTG GGC AAG CGG
Leu Pro Gly Met Met Arg Leu Tyr Ser Glu Phe Leu Gly Lys Arg
```

```
CCA TGG TTT GCA GGG GAC AAG ATC ACC TTT GTG GAT TTC ATT GCT
Pro Trp Phe Ala Gly Asp Lys Ile Thr Phe Val Asp Phe Ile Ala

TAC GAT GTT CTT GAG AGG AAC CAA GTG TTT GAG GCC ACG TGC CTG
Tyr Asp Val Leu Glu Arg Asn Gln Val Phe Glu Ala Thr Cys Leu

GAC GCG TTC CCA AAC CTG AAG GAT TTC ATA GCG CGC TTT GAG GGC
Asp Ala Phe Pro Asn Leu Lys Asp Phe Ile Ala Arg Phe Glu Gly

CTG AAG AAG ATC TCC GAC TAC ATG AAG TCC AGC CGC TTC CTC CCA
Leu Lys Lys Ile Ser Asp Tyr Met Lys Ser Ser Arg Phe Leu Pro

AGA CCT CTG TTC ACA AAG ATG GCT ATT TGG GGC AGC AAG TAG GAC
Arg Pro Leu Phe Thr Lys Met Ala Ile Trp Gly Ser Lys End Asp

CCT GAC AGG TGG GCT TTA GGA GAA AGA TAC CAA ATC TCC TGG GTT
Pro Asp Arg Trp Ala Leu Gly Glu Arg Tyr Gln Ile Ser Trp Val

TGC CAA GAG CCC TAA GGA GCG GGC AGG ATT CCT GAG CCC CAG AGC
Cys Gln Glu Pro End Gly Ala Gly Arg Ile Pro Glu Pro Gln Ser

CAT GTT TTC TTC CTT CCT TCC ATT CCA GTC CCC AAG CCT TAC CAG
His Val Phe Phe Leu Pro Ser Ile Pro Val Pro Lys Pro Tyr Gln

CTC TCA TTT TTT GGT CAT CAA ATT CCT GCC AAA CAC AGG CTC TTA
Leu Ser Phe Phe Gly His Gln Ile Pro Ala Lys His Arg Leu Leu

AAA GCC CTA GCA ACT CCT TTC CAT TAG CAA AAT AGC CTT CTA AAG
Lys Ala Leu Ala Thr Pro Phe His End Gln Asn Ser Leu Leu Lys

TTA AAG TGC CCC GCC CCC ACC CCT CGA GCT CAT GTG ATT GGA TAG
Leu Lys Cys Pro Ala Pro Thr Pro Arg Ala His Val Ile Gly End

TTG GCT CCC AAC ATG TGA TTA TTT TGG GCA GGT CAG GCT CCC CGG
Leu Ala Pro Asn Met End Leu Phe Trp Ala Gly Gln Ala Pro Arg

CAG ATG GGG TCT ATC TGG AGA CAG TAG ATT GCT AGC AGC TTT GAC
Gln Met Gly Ser Ile Trp Arg Gln End Ile Ala Ser Ser Phe Asp

CAC CGT AGC CAA GCC CCT CTT CTT GCT GTT TCC CGA GAC TAG CTA
His Arg Ser Gln Ala Pro Leu Leu Ala Val Ser Arg Asp End Leu

TGA GCA AGG TGT GCT GTG TCC CCA GCA CTT GTC ACT GCC TCT GTA
End Ala Arg Cys Ala Val Ser Pro Ala Leu Val Thr Ala Ser Val
```

```
        113 0                              1 150                              11
ACC CGC TCC TAC CGC TCT TTC TTC CTG CTG CTG TGA GCT GTA CCT
Thr Arg Ser Tyr Arg Ser Phe Phe Leu Leu Leu End Ala Val Pro

        70                               119 0                              1 210
CCT GAC CAC AAA CCA GAA TAA ATC ATT CTC CCC TTA AAA AAA AAA
Pro Asp His Lys Pro Glu End Ile Ile Leu Pro Leu Lys Lys Lys

AAA AAA AAA A
Lys Lys Lys
```

sowie deren Varianten oder Mutanten, die für Polypeptide mit Glutathion-S-Transferase-Aktivität kodieren.

13. Ein rekombinantes DNA-Molekül gemäss einem der Ansprüche 1, 2 oder 8, dadurch gekennzeichnet, dass besagte Gen-Sequenz entweder aus genomischer DNA oder aus einer cDNA besteht.

14. Ein rekombinantes DNA-Molekül gemäss einem der Ansprüche 1, 2 oder 8, dadurch gekennzeichnet, dass besagte Gen-Sequenz sowohl aus genomischer DNA, als auch aus cDNA zusammengesetzt ist.

15. Ein rekombinantes DNA-Molekül gemäss einem der Ansprüche 1, 2 oder 8, dadurch gekennzeichnet, dass die Gen-Sequenz aus Genfragmenten von mehreren Organismen, die verschiedenen Gattungen angehören, zusammengesetzt ist.

16. Ein rekombinantes DNA-Molekül gemäss einem der Ansprüche 1, 2 oder 8, dadurch gekennzeichnet, dass die Gen-Sequenz aus Genfragmenten von mehr als einem Stamm, einer Varietät oder einer Spezies des gleichen Organismus zusammengesetzt ist.

17. Ein rekombinantes DNA-Molekül gemäss einem der Ansprüche 1, 2 oder 8, dadurch gekennzeichnet, dass die Gen-Sequenz aus Anteilen von mehr als einem Gen des selben Organismus zusammengesetzt ist.

18. Ein rekombinantes DNA-Molekül, dadurch gekennzeichnet, dass es aus einer Gen-Sequenz besteht, die für ein Glutathion S-Transferase Polypeptid kodiert und die in operabler Weise mit einem pflanzlichen Promotor verknüpft ist.

19. Ein rekombinantes DNA-Molekül gemäss Anspruch 18, dadurch gekennzeichnet, dass die Gen-Sequenz heterologen Ursprungs ist in Bezug auf den pflanzlichen Promotor.

20. Ein rekombinantes DNA-Molekül gemäss Anspruch 18, dadurch gekennzeichnet, dass die Gen-Sequenz in pflanzlichen Zellen exprimiert wird.

21. Ein rekombinantes DNA-Molekül gemäss Anspruch 19, dadurch gekennzeichnet, dass die Gen-Sequenz in pflanzlichen Zellen exprimiert wird.

22. Ein rekombinantes DNA-Molekül gemäss Anspruch 20, dadurch gekennzeichnet, das es sich bei den pflanzlichen Zellen um Zellen von Tabak, Sojabohnen oder Baumwolle handelt.

23. Ein rekombinantes DNA-Molekül gemäss Anspruch 20, dadurch gekennzeichnet, dass die pflanzlichen Zellen Teile einer ganzen Pflanze sind.

24. Ein rekombinantes DNA-Molekül gemäss Anspruch 18, dadurch gekennzeichnet, dass es sich bei dem pflanzlichen Promotor um einen Promotor, ausgewählt aus der Gruppe der nos-, ocs- und CaMV-Promotoren, handelt.

25. Ein rekombinantes DNA-Molekül gemäss Anspruch 18, dadurch gekennzeichnet, dass es sich bei besagtem pflanzlichen Promotor um den Promotor der kleinen Untereinheit der Ribulose-bis-phosphat-Carboxylase der Sojabohne oder um den Promotor des Chlorophyll-a/b-Bindungsproteins handelt.

26. Ein DNA-Transfer-Vektor, dadurch gekennzeichnet, dass er das rekombinante DNA-Molekül genmäss einem der Ansprüche 1, 2 oder 8 bis 13 enthält.

27. Ein DNA-Expressions-Vektor, dadurch gekennzeichnet, dass er ein rekombinantes DNA-Molekül gemäss einem der Ansprüche 1, 2 oder 8 bis 13 enthält.

28. Eine Wirtszelle, dadurch gekennzeichnet, dass sie einen DNA-Transfer-Vektor gemäss Anspruch 26 enthält.

29. Eine Wirtszelle, dadurch gekennzeichnet, dass sie einen DNA-Expressions-Vektor gemäss Anspruch 27 enthält.

30. Eine Wirtszelle gemäss einem der Ansprüche 28 oder 29, dadurch gekennzeichnet, dass es sich dabei um einen Mikroorganismus handelt.

31. Eine Wirtszelle gemäss einem der Ansprüche 28 oder 29, dadurch gekennzeichnet, dass es sich dabei um eine pflanzliche Zelle handelt.

32. Eine Wirtszelle, dadurch gekennzeichnet, dass sie ein rekombinantes DNA-Molekül gemäss einem der Ansprüche 1, 2 oder 8 bis 13 enthält.

33. Eine Wirtszelle gemäss Anspruch 32, dadurch gekennzeichnet, dass es sich dabei um einen Mikroorganismus handelt.

34. Eine Wirtszelle gemäss Anspruch 32, dadurch gekennzeichnet, dass es sich dabei um eine pflanzliche Zelle handelt.

35. Eine Pflanze und deren Samen, dadurch gekennzeichnet, dass sie pflanzliche Zellen gemäss Anspruch 34 enthalten.

36. Die Nachkommenschaft einer Pflanze, dadurch gekennzeichnet, dass besagte Nachkommen aus pflanzlichen Zellen gemäss Anspruch 35 regeneriert worden sind und, dass besagte Nachkommenschaft Mutanten und Varianten miteinschliesst.

37. Eine Pflanze gemäss Anspruch 35, ausgewählt aus der Gruppe bestehend aus Lotus, Medicago, Onobrychis, Trifolium, Trigonella, Citrus, Linum, Manihot, Daucus, Arabidopsis, Brassica, Raphanus, Sinapis, Atropa, Capsicum, Datura, Hyoscyamus, Lycopersicon, Nicotiana, Solanum, Petunia, Majorana, Cichorium, Helianthus, Lactuca, Asparagus, Antirrhinum, Panicum, Pennisetum, Ranunculus, Salpiglossis, Glycine, Gossypium, Malus, Prunus, Rosa, Populus, Allium, Lilium, Narcissus, Ananas, Arachis, Phaseolus und Pisum.

38. Eine Pflanze gemäss Anspruch 36, ausgewählt aus der Gruppe bestehend aus Lolium, Zea, Triticum, Sorghum und Bromus.

39. Der Samen von Pflanzen gemäss Anspruch 35.

40. Der Samen von Pflanzen gemäss Anspruch 36.

41. Die Nachkommenschaft einer Pflanze, dadurch gekennzeichnet, dass besagte Nachkommen aus pflanzlichen Zellen gemäss Anspruch 37 regeneriert worden sind und dass besagte Nachkommenschaft Mutanten und Varianten miteinschliesst.

42. Die Nachkommenschaft von Pflanzen, dadurch gekennzeichnet, dass besagte Nachkommen aus pflanzlichen Zellen gemäss Anspruch 38 regeneriert worden sind und, dass besagte Nachkommenschaft Mutanten und Varianten miteinschliesst.

43. Verfahren zur Herstellung einer Herbizid-toleranten pflanzlichen Zelle, dadurch gekennzeichnet, dass man eine pflanzliche Zelle mit einem rekombinanten DNA-Molekül gemäss einem der Ansprüche 1, 2 oder 8 bis 13 transformiert.

44. Verfahren zur Herstellung Herbizid-toleranter Pflanzen, dadurch gekennzeichnet, dass man Pflanzen aus pflanzlichen Zellen gemäss Anspruch 34 regeneriert.

45. Verfahren zur selektiven Bekämpfung Herbizid-sensitiver Pflanzen in einer Mischpopulation bestehend aus besagten Herbizid-sensitiven Pflanzen und Herbizid-toleranten Pflanzen gemäss einem der Ansprüche 35 oder 36, dadurch gekennzeichnet, dass man die Mischpopulation mit einer für die Bekämpfung der Herbizid-sensitiven Pflanzen wirksamen Menge des Herbizids in Kontakt bringt, die ausreicht, das Wachstum oder die Entwicklung besagter Herbizid-sensitiver Pflanzen zu beeinträchtigen.

46. Verfahren zur selektiven Bekämpfung Herbizid-sensitiver Pflanzen in einer Mischpopulation bestehend aus besagten Herbizid-sensitiven Pflanzen und Herbizid-toleranten Pflanzen gemäss einem der Ansprüche 35 oder 36, dadurch gekennzeichnet, dass man die Mischpopulation mit einer für die Bekämpfung der Herbizid-sensitiven Pflanzen wirksamen Menge des Herbizids und mit einem Sensibilisator in Kontakt bringt, die ausreicht, das Wachstum oder die Entwicklung besagter Herbizid-sensitiver Pflanzen zu beeinträchtigen.

47. Das vir-Plasmid pCIB542.

48. Eine Pflanze mit Pflanzenzellen, die mit Agrobakterium tumefaciens transformiert wurden, welches das Plasmid pCIB542 und ein T-DNA-Plasmid enthält.

49. Eine Pflanze gemäss Anspruch 48, ausgewählt aus der Gruppe bestehend aus Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine,

0256223

<u>Lolium, Zea, Triticum, Sorghum, Ipomoea, Passiflora, Cyclamen,</u>
<u>Malus, Prunus, Rosa, Rubus, Populus, Santalum, Allium, Lilium,</u>
<u>Narcissus, Ananas, Arachis, Phaseolus und Pisum.</u>

50. Eine Pflanze und deren Samen, dadurch gekennzeichnet, dass sie
aus pflanzlichen Zellen gemäss Anspruch 49 regeneriert worden ist.

51. Die Nachkommenschaft einer Pflanze, dadurch gekennzeichnet,
dass besagte Nachkommen aus pflanzlichen Zellen gemäss Anspruch 49
regeneriert worden sind und, dass besagte Nachkommenschaft Mutanten
und Varianten miteinschliesst.

FO 7.5/WB/hpw*/ga*

Fig. 1

p CIB 710

_Fig. 2_

*Fig. 3*

Fig. 4A

# RECOMBINATIONS METHODE

Ap$^r$

col E1
PLASMID

GENOMISCHE
DNA

Ap$^r$ Tc$^r$
BEI 42°C
IN

polA 1　　　sup F 183

E.coli
(SR 43)

c DNA

IncP 1
PLASMID

Tc$^r$

Ap$^r$

COINTEGRIERTES
PLASMID

Tc$^r$

VOLLSTÄNDIGES
GST- GEN

*Fig. 4* B

## IN VITRO LIGATIONS METHODE

Ap$^r$

GST GENOMISCHE DNA

Pst I

Hind III

Hinf I

GST c DNA

Hinf I

Hinf I

Hind III

SCHNEIDEN MIT EINEM Hinf I-ISOLAT

SCHNEIDEN MIT Hinf I, BEHANDELN MIT ALKALISCHER PHOSPHATASE AUS KÄLBERDARM.

Hinf I

Hinf I

VERKNÜPFEN

VOLLSTÄNDIGES GST-GEN

Pst I

Hind III

Hinf I   Hind III   Bgl I   Hinf I

*Fig. 4*C

TABAK NICHT-TRANSFORMIERT

Fig. 5

## TABAK  TRANSFORMIERT

FLUORESZENZ  INDUKTION

$10^{-5}$ M  ATRAZIN

RELATIVE FLUORESZENZ

LICHT AN                    *Fig: 6*                 ZEIT (SEKUNDEN)

Fig. 7a

*Fig. 7b*

Fig. 7c

Fig. 3d

Fig. 7e

Fig. 7f

**Fig. 7g**

# EUROPÄISCHER TEILRECHERCHENBERICHT,

der nach Regel 45 des Europäischen Patent-übereinkommens für das weitere Verfahren als europäischer Recherchenbericht gilt

Europäisches Patentamt

**0256223**
Nummer der Anmeldung

## EINSCHLÄGIGE DOKUMENTE

EP 87107137.9

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A2 - 0 154 204 (MOLECULAR GENETICS, INC.) <br><br> * Zusammenfassung; Ansprüche 1-3,7-9 * <br><br> -- | 1,2,4, 13,35, 36,38, 43 | C 12 N 15/00 <br> C 07 H 21/04 <br> C 12 N 5/00 <br> A 01 H 1/00 <br> C 12 N 9/10 |
| X | WO - A1 - 86/02 097 (THE GENERAL HOSPITAL CORPORATION) <br><br> * Seite 1, Ansprüche 11-14 * <br><br> -- | 1,4,18 | |
| D,A | PHYTOCHEMISTRY, vol. 21, no. 12, 1982 (Oxford, GB) <br><br> H. RENNENBERG "Glutathione Metabolism and Possible Biological Roles in Higher Plants" Seiten 2771-2781 <br><br> * Gesamt * <br><br> ---- | | |

### RECHERCHIERTE SACHGEBIETE (Int. Cl.4)

C 12 N
C 07 H
A 01 H

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-38, 41-51

Unvollständig recherchierte Patentansprüche: --

Nicht recherchierte Patentansprüche: 39,40 (Artikel 53(b) EPÜ)

Grund für die Beschränkung der Recherche:

| Recherchenort | Abschlußdatum der Recherche | Prüfer |
|---|---|---|
| WIEN | 25-08-1987 | WOLF |

EPA Form 1505.1  08.82